# EUROPÄISCHE PATENTSCHRIFT

(11) **EP 1 937 673 B1**
(45) Veröffentlichungstag und Bekanntmachung des Hinweises auf die Patenterteilung: **21.10.2015**
(21) Anmeldenummer: 06792327.6
(22) Anmeldetag: 30.09.2006
(51) Int. Cl.: C07D 417/14, C07D 413/14

(54) **IMIDAZOLDERIVATE ALS INHIBITOREN VON TAFI-A**
IMIDAZOLE DERIVATIVES AS INHIBITORS OF TAFIA
DERIVES IMIDAZOLE SERVANT D'INHIBITEURS DE TAFI-A

(30) Priorität: 15.10.2005 DE 102005049385
(43) Veröffentlichungstag der Anmeldung: 02.07.2008
(73) Patentinhaber: SANOFI, 75008 Paris (FR)
(72) Erfinder: KALLUS, Christopher, 65926 Frankfurt am Main (DE); HEITSCH, Holger, 65926 Frankfurt am Main (DE); WEHNER, Volkmar, 65926 Frankfurt am Main (DE)
(74) Vertreter: Then, Johann
(86) Internationale Anmeldenummer: PCT/EP2006/009500
(87) Internationale Veröffentlichungsnummer: WO 2007/045339

(56) Entgegenhaltungen:
- WO-A-02/14285
- WO-A-03/013526
- WO-A1-03/061653

## Beschreibung

Die Erfindung betrifft neue Verbindungen der Formel I, die das Enzym TAFIa (aktivierter Thrombin-aktivierbarer Fibrinolyse Inhibitor) inhibieren, Verfahren zu ihrer Herstellung und Verwendung derselben als Arzneimittel.

Das Enzym TAFIa entsteht beispielsweise durch Thrombinaktivierung aus dem Thrombin-aktivierbaren-Fibrinolyse-Inhibitor-Zymogen (TAFI). Das Enzym TAFI wird auch als Plasma Procarboxypeptidase B, Procarboxypeptidase U oder als Procarboxypeptidase R bezeichnet und ist ein Carboxypeptidase B ähnliches Proenzym (L. Bajzar, Arterioscler. Thromb. Vasc. Biol. 2000, Seiten 2511 - 2518).

Während einer Gerinnselbildung wird Thrombin als das Endprodukt der Koagulationskaskade generiert und induziert die Konversion von löslichem Plasmafibrinogen zu einer unlöslichen Fibrinmatrix. Gleichzeitig aktiviert Thrombin den endogenen Fibrinolyse-Inhibitor TAFI. Aktiviertes TAFI (TAFIa) entsteht also während der Thrombusbildung und der Lyse aus dem Zymogen TAFI unter der Einwirkung von Thrombin; Thrombomodulin im Komplex mit Thrombin verstärkt diesen Effekt etwa 1250 fach. TAFIa spaltet basische Aminosäuren am carboxy-Ende des Fibrins. Der Verlust der carboxy-terminalen Lysine als Bindestellen für Plasminogen führt dann zu einer Inhibition der Fibrinolyse. Effektive Inhibitoren von TAFIa verhindern den Verlust dieser hoch affinen Lysin-Bindungsstellen für Plasminogen und unterstützen auf diese Weise die endogene Fibrinolyse durch Plasmin: TAFIa Inhibitoren wirken profibrinolytisch.

Um die Hämostase im Blut aufrechtzuerhalten, haben sich Mechanismen ausgebildet, die zur Blutgerinnung und zur Auflösung von Gerinnseln führen; diese stehen in einem Gleichgewicht. Wenn ein gestörtes Gleichgewicht die Koagulation begünstigt, entsteht Fibrin in größeren Mengen, so dass pathologische Vorgänge der Thrombusbildung zu schweren Krankheitsbildern im Menschen führen können.

Genauso wie eine überschießende Koagulation zu schwerwiegenden thrombotisch bedingten Krankheitsbildern führen kann, besitzt eine antithrombotische Behandlung das Risiko von nicht erwünschten Blutungen durch eine Störung der Bildung eines notwendigen hämostatischen Pfropfs. Die Hemmung von TAFIa verstärkt - ohne die Koagulation und die Plättchenaggregation zu beeinflussen - die endogene Fibrinolyse d.h. das gestörte Gleichgewicht wird zugunsten der Fibrinolyse verschoben. So kann sowohl dem Aufbau eines klinisch relevanten Thrombus entgegengewirkt, als auch die Lyse eines schon bestehenden Gerinnsels verstärkt werden. Andererseits wird der Aufbau eines hämostatischen Pfropfs nicht beeinträchtigt, sodass eine Blutungsdiathese eher nicht zu erwarten ist (Bouma et al., J. Thrombosis and Haemostasis, 1, 2003, Seiten 1566 - 1574).

Inhibitoren von TAFIa sind bereits in den Internationalen Anmeldungen WO02/14285, WO03/013526 und WO03/061653 beschrieben worden.

Die erfindungsgemäßen TAFIa Inhibitoren eignen sich für eine prophylaktische als auch für eine therapeutische Anwendung am Menschen, die an Erkrankungen leiden, die mit Thrombosen, Embolien, Hyperkoagulabilität oder fibrotischen Veränderungen einhergehen. Sie können zur Primär- und Sekundär-Prevention eingesetzt werden und eignen sich sowohl für eine akute als auch für eine Langzeittherapie.

Die Erfindung betrifft daher eine Verbindung der Formel I 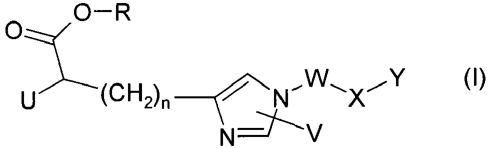 und/oder alle stereoisomeren Formen der Verbindung der Formel I und/oder Gemische dieser Formen in jedem Verhältnis, und/oder ein physiologisch verträgliches Salz der Verbindung der Formel I, wobei
U für 1) -(C₁-C₄)-Alkylen-Z und
U und Z zusammen den Rest 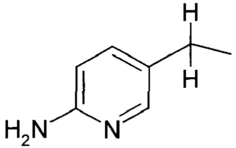 darstellen und der Pyridylteil im Rest unsubstituiert oder durch Methyl oder Ethyl substituiert ist,
- n: für die ganze Zahl Null steht,
- R: für Wasserstoffatom oder -(C₁-C₄)-Alkyl steht,
- V: für
1) Wasserstoffatom,
2) -(C₁-C₃)-Alkyl oder
3) Fluor, Chlor oder Brom, steht,
- W: für -(C₁-C₃)-Alkylen steht,
- X: für einen aromatischen fünf- bis dreizehngliedrigen Heterocyclus steht, wobei Heterocyclus ausgewählt ist aus der Gruppe Isoxazolyl, 1,2,3-Oxadiazolyl, 1,2,4-Oxadiazolyl, 1,2,5-Oxadiazolyl, 1,3,4-Oxadiazolyl, Oxazolyl, 1,2,3-Thiadiazolyl, 1,2,4-Thiadiazolyl, 1,2,5-Thiadiazolyl, 1,3,4-Thiadiazolyl, Thienopyridin oder Thienyl, und Heterocyclus unsubstituiert oder ein-, zweioder dreifach unabhängig voneinander durch R1 substituiert ist,
Y für
1) -(C₃-C₆)-Cycloalkyl, wobei Cycloalkyl unsubstituiert oder ein-, zwei- oder dreifach unabhängig voneinander durch R1 substituiert ist,
2) -(C₆-C₁₄)-Aryl, wobei Aryl ausgewählt ist aus der Gruppe Indanyl, Naphthyl, Phenyl oder Tetrahydronaphthalenyl, wobei Aryl unsubstituiert oder ein-, zwei- oder dreifach unabhängig voneinander durch R1 substituiert ist, oder
3) Heterocyclus steht, wobei Heterocyclus ausgewählt ist aus der Gruppe 2,3-Dihydro-benzo[1,4]dioxin, Isoxazolyl, 1,2,3-Oxadiazolyl, 1,2,4-Oxadiazolyl, 1,2,5-Oxadiazolyl, 1,3,4-Oxadiazolyl, Oxazolyl, 1,2,3-Thiadiazolyl, 1,2,4-Thiadiazolyl, 1,2,5-Thiadiazolyl, 1,3,4-Thiadiazolyl, Thienopyridin oder Thienyl, und Heterocyclus unsubstituiert oder ein-, zwei- oder dreifach unabhängig voneinander durch R1 substituiert ist, und
- R1 für: Fluor, Chlor, Brom, -(C₁-C₄)-Alkyl, -(C₀-C₄)-Alkylen-Phenyl, -O-CH₃, -O-(C₁-C₄)-Alkylen-Phenyl, wobei Phenyl unsubstituiert oder ein-, oder zweifach durch Fluor, Chlor, Brom oder-O-(C₁-C₄)-Alkyl substituiert ist, -(C₃-C₆)-Cycloalkyl oder -CF₃ steht.

Ein weiterer Gegenstand der Erfindung sind Verbindungen der Formel I aus der Reihe
3-(6-Amino-pyridin-3-yl)-2-{1-[5-(5-chlor-thiophen-2-yl)-isoxazol-3-ylmethyl]-1H-imidazol-4-yl}-propionsäure,
3-(6-Amino-pyridin-3-yl)-2-[1-(5-phenyl-isoxazol-3-ylmethyl)-1 H-imidazol-4-yl]-propionsäure,
3-(6-Amino-pyridin-3-yl)-2-{1-[5-(4-methoxy-phenyl)-isoxazol-3-ylmethyl]-1H-imidazol-4-yl}-propionsäure,
3-(6-Amino-pyridin-3-yl)-2-{1-[5-(5-chlor-thiophen-2-yl)-[1,3,4]thiadiazol-2-ylmethyl]-1 H-imidazol-4-yl}- propionsäure,
3-(6-Amino-pyridin-3-yl)-2-{1-[5-(4-chlor-phenyl)-[1,3,4]thiadiazol-2-ylmethyl]-1H-imidazol-4-yl}- propionsäure,
3-(6-Amino-pyridin-3-yl)-2-{1-[5-(4-trifluormethyl-phenyl)-[1,2,4]oxadiazol-3-ylmethyl]-1 H-imidazol-4-yl}- propionsäure,
3-(6-Amino-pyridin-3-yl)-2-{1-[5-(3-trifluormethyl-phenyl)-[1,2,4]oxadiazol-3-ylmethyl]-1 H-imidazol-4-yl}- propionsäure,
3-(6-Amino-pyridin-3-yl)-2-[1-(5-cyclopropyl-[1,3,4]thiadiazol-2-ylmethyl)-1 H-imidazol-4-yl]-propionsäure,
3-(6-Amino-pyridin-3-yl)-2-[1-(5-phenyl-[1,2,4]oxadiazol-3-ylmethyl)-1 H-imidazol-4-yl]-propionsäure,
3-(6-Amino-pyridin-3-yl)-2-{1-[3-(3,4-dimethoxy-phenyl)-[1,2,4]oxadiazol-5-ylmethyl]-1 H-imidazol-4-yl}-propionsäure,
3-(6-Amino-pyridin-3-yl)-2-{1-[3-(4-methoxy-phenyl)-[1,2,4]oxadiazol-5-ylmethyl]-1H-imidazol-4-yl}-propionsäure,
3-(6-Amino-pyridin-3-yl)-2-{1-[3-(4'-isopropyl-phenyl)-[1,2,4]oxadiazol-5-ylmethyl]-1H-imidazol-4-yl}-propionsäure,
3-(6-Amino-pyridin-3-yl)-2-{1-[3-(4'-tert-butyl-phenyl)-[1,2,4]oxadiazol-5-ylmethyl]-1H-imidazol-4-yl}-propionsäure,
3-(6-Amino-pyridin-3-yl)-2-{1-[5-(3,4-dichlor-phenyl)-isoxazol-3-ylmethyl]-1H-imidazol-4-yl}-propionsäure,
3-(6-Amino-pyridin-3-yl)-2-{1-[3-(4-methoxy-phenyl)-[1,2,4]oxadiazol-5-ylmethyl]-1H-imidazol-4-yl}-propionsäure,
3-(6-Amino-pyridin-3-yl)-2-[1-(5-phenyl-[1,3,4]thiadiazol-2-ylmethyl)-1 H-imidazol-4-yl]-propionsäure,
3-(6-Amino-pyridin-3-yl)-2-{1-[5-(4-methoxy-phenyl)-[1,3,4]thiadiazol-2-ylmethyl]-1H-imidazol-4-yl}-propionsäure,
3-(6-Amino-pyridin-3-yl)-2-{1-[5-(3,5-dimethyl-isoxazol-4-yl)-[1,2,4]oxadiazol-3-ylmethyl]-1H-imidazol-4-yl}-propionsäure,
3-(6-Amino-pyridin-3-yl)-2-[1-(5-thiophen-2-yl-isoxazol-3-ylmethyl)-1 H-imidazol-4-yl]-propionsäure,
3-(6-Amino-pyridin-3-yl)-2-(1-{3-[4-(4-chlor-benzyloxy)-phenyl]-[1,2,4]oxadiazol-5-ylmethyl}-1 H-imidazol-4-yl)-propionsäure,
3-(6-Amino-pyridin-3-yl)-2-[1-(4-phenyl-5-trifluormethyl-thiophen-2-ylmethyl)-1H-imidazol-4-yl]-propionsäure,
3-(6-Amino-pyridin-3-yl)-2-{1-[5-(4-brom-phenyl)-isoxazol-3-ylmethyl]-1H-imidazol-4-yl}-propionsäure,
3-(6-Amino-pyridin-3-yl)-2-[1-(5-p-tolyl-isoxazol-3-ylmethyl)-1H-imidazol-4-yl]-propionsäure,
3-(6-Amino-pyridin-3-yl)-2-(1-{2-[5-(5-chlor-thiophen-2-yl)-isoxazol-3-yl]-ethyl}-1H-imidazol-4-yl)-propionsäure,
3-(6-Amino-pyridin-3-yl)-2-{1-[5-(4-isobutyl-phenyl)-isoxazol-3-ylmethyl]-1H-imidazol-4-yl}-propionsäure,
3-(6-Amino-pyridin-3-yl)-2-[1-(5-cyclopentyl-[1,3,4]thiadiazol-2-ylmethyl)-1H-imidazol-4-yl]-propionsäure,
3-(6-Amino-pyridin-3-yl)-2-[1-(5-cyclobutyl-[1,3,4]thiadiazol-2-ylmethyl)-1H-imidazol-4-yl]-propionsäure,
3-(6-Amino-pyridin-3-yl)-2-[1-(5-cyclopropyl-isoxazol-3-ylmethyl)-1 H-imidazol-4-yl]-propionsäure,
3-(6-Amino-pyridin-3-yl)-2-[1-(5-cyclohexyl-[1,3,4]thiadiazol-2-ylmethyl)-1 H-imidazol-4-yl]-propionsäure,
3-(6-Amino-pyridin-3-yl)-2-[1-(5-cyclobutyl-isoxazol-3-ylmethyl)-1 H-imidazol-4-yl]-propionsäure,
3-(6-Amino-pyridin-3-yl)-2-{1-[5-(4-fluor-phenyl)-isoxazol-3-ylmethyl]-1H-imidazol-4-yl}-propionsäure,
3-(6-Amino-pyridin-3-yl)-2-{1-[5-(4-benzyl-phenyl)-isoxazol-3-ylmethyl]-1H-imidazol-4-yl}-propionsäure,
3-(6-Amino-pyridin-3-yl)-2-{1-[5-(4-tert-butyl-phenyl)-isoxazol-3-ylmethyl]-1H-imidazol-4-yl}-propionsäure,
3-(6-Amino-pyridin-3-yl)-2-{1-[5-(4-tert-butyl-2,6-dimethyl-phenyl)-isoxazol-3-ylmethyl]-1 H-imidazol-4-yl}-propionsäure,
3-(6-Amino-pyridin-3-yl)-2-{1-[5-(2-chlor-phenyl)-isoxazol-3-ylmethyl]-1H-imidazol-4-yl}-propionsäure,
3-(6-Amino-pyridin-3-yl)-2-{1-[5-(4-sec-butyl-phenyl)-isoxazol-3-ylmethyl]-1H-imidazol-4-yl}-propionsäure,
3-(6-Amino-pyridin-3-yl)-2-[1-(5-indan-5-yl-isoxazol-3-ylmethyl)-1H-imidazol-4-yl]-propionsäure,
3-(6-Amino-pyridin-3-yl)-2-{1-[5-(4-cyclopentyl-phenyl)-isoxazol-3-ylmethyl]-1H-imidazol-4-yl}-propionsäure,
3-(6-Amino-pyridin-3-yl)-2-{1-[5-(4-isopropyl-phenyl)-isoxazol-3-ylmethyl]-1H-imidazol-4-yl}-propionsäure,
3-(6-Amino-pyridin-3-yl)-2-{1-[5-(4-butyl-phenyl)-isoxazol-3-ylmethyl]-1H-imidazol-4-yl}-propionsäure,
3-(6-Amino-pyridin-3-yl)-2-{1-[5-(4-cyclohexyl-phenyl)-isoxazol-3-ylmethyl]-1H-imidazol-4-yl}-propionsäure,
3-(6-Amino-pyridin-3-yl)-2-{1-[5-(5,6,7,8-tetrahydro-naphthalen-2-yl)-isoxazol-3-ylmethyl]-1H-imidazol-4-yl}-propionsäure,
3-(6-Amino-pyridin-3-yl)-2-{1-[5-(4-propyl-phenyl)-isoxazol-3-ylmethyl]-1H-imidazol-4-yl}-propionsäure,
3-(6-Amino-pyridin-3-yl)-2-{1-[5-(4-phenethyl-phenyl)-isoxazol-3-ylmethyl]-1H-imidazol-4-yl}-propionsäure,
3-(6-Amino-pyridin-3-yl)-2-{1-[5-(2,3-dihydro-benzo[1,4]dioxin-6-yl)-isoxazol-3-ylmethyl]-1H-imidazol-4-yl}-propionsäure,
(S)-3-(6-Amino-pyridin-3-yl)-2-{1-[5-(5-chlor-thiophen-2-yl)-isoxazol-3-ylmethyl]-1H-imidazol-4-yl}-propionsäureethylester,
(R)-3-(6-Amino-pyridin-3-yl)-2-{1-[5-(5-chlor-thiophen-2-yl)-isoxazol-3-ylmethyl]-1H-imidazol-4-yl}-propionsäureethylester,
(S)-3-(6-Amino-pyridin-3-yl)-2-{1-[5-(5-chlor-thiophen-2-yl)-isoxazol-3-ylmethyl]-1H-imidazol-4-yl}-propionsäure,
(R)-3-(6-Amino-pyridin-3-yl)-2-{1-[5-(5-chlor-thiophen-2-yl)-isoxazol-3-ylmethyl]-1H-imidazol-4-yl}-propionsäure oder
3-(6-Amino-5-methyl-pyridin-3-yl)-2-[1-(5-cyclopropyl-[1,3,4]thiadiazol-2-ylmethyl)-1 H-imidazol-4-yl]-propionsäure.

Unter dem Begriff "(C₁-C₄)-Alkyl"werden Kohlenwasserstoff-reste verstanden, deren Kohlenstoffkette geradkettig oder verzweigt ist und 1 bis 4 Kohlenstoffatome enthält, beispielsweise Methyl, Ethyl, Propyl, Iso-Propyl, Butyl, Iso-Butyl oder tertiär-Butyl. Unter dem Begriff "-(C₀-C₄)-Alkylen" werden Kohlenwasserstoffreste verstanden, deren Kohlenstoffkette geradkettig oder verzweigt ist und 1 bis 4 Kohlenstoffatome enthält, beispielsweise Methylen, Ethylen, Propylen, Iso-Propylen, Iso-Butylen, Butylen oder tertiär-Butylen. "-C₀-Alkylen" ist eine kovalente Bindung.

Unter dem Begriff "-(C₁-C₄)-Alkylen" werden Kohlenwasserstoffreste verstanden, deren Kohlenstoffkette geradkettig oder verzweigt ist und 1 bis 4 Kohlenstoffatome enthält, beispielsweise Methylen (-CH₂-), Ethylen (-CH₂-CH₂-), Propylen (-CH₂-CH₂-CH₂-), Iso-Propylen, Iso-Butylen, Butylen oder tertiär-Butylen.

Einige dieser Alkylenreste für den Rest W verbinden das N-Atom im Imidazolring mit dem Rest X.

Unter dem Begriff "(C₃-C₁₂)-Cycloalkyl" werden Reste verstanden wie z. B. die Monocyclen Cyclopropan, Cyclobutan, Cyclopentan, Cyclohexan.

Unter dem Begriff "-(C₆-C₁₄)-Aryl" werden aromatische Kohlenstoffreste verstanden mit 6 bis 14 Kohlenstoffatomen im Ring. Beispiele für -(C₆-C₁₄)-Aryl sind die Reste Indanyl, Naphthyl, zum Beispiel 1-Naphthyl, 2-Naphthyl, Phenyl oder Tetrahydronaphthalenyl. Indanyl-, Tetrahydronaphthalenyl- und insbesondere Phenylreste sind bevorzugte Arylreste.

Unter dem Begriff "aromatischen fünf- bis dreizehngliedrigen Heterocyclus" werden Ringsysteme verstanden mit 5 bis 13 Kohlenstoffatomen, die ein, zwei oder drei gleiche oder verschiedene Heteroatome aus der Reihe Sauerstoff, Stickstoff oder Schwefel enthalten können. Beispiele für diese Ringsysteme sind die Reste 2,3-Dihydro-benzo[1,4]dioxin, Isoxazolyl, 1,2,3-Oxadiazolyl, 1,2,4-Oxadiazolyl, 1,2,5-Oxadiazolyl, 1,3,4-Oxadiazolyl, Oxazolyl, 1,2,3-Thiadiazolyl, 1,2,4-Thiadiazolyl, 1,2,5-Thiadiazolyl, 1,3,4-Thiadiazolyl, Thienyl und Thienothiazolyl,.

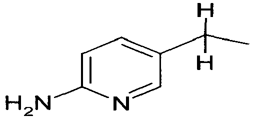

Unter dem Rest wird das 5-Methylen-pyridin-2-ylamin verstanden, wobei das Pyridin über den Methylenrest an den Rest der Verbindung der Formel I gebunden ist.

Bevorzugte Het-Ringe sind die Reste Isoxazolyl, 1,2,3-Oxadiazolyl, 1,2,4-Oxadia-zolyl, 1,2,5-Oxadiazolyl, 1,3,4-Oxadiazolyl, Oxazolyl, 1,2,3-Thiadiazolyl, 1,2,4-Thiadiazolyl, 1,2,5-Thiadiazolyl, 1,3,4-Thiadiazolyl und Thienopyridin.

Die erfindungsgemäßen Verbindungen können nach wohlbekannten Verfahren oder nach hier beschriebenen Verfahren hergestellt werden.

Funktionelle Gruppen der verwendeten Intermediate, beispielsweise Amino- oder Carboxylgruppen wie der -COOR-Rest in der Verbindung der Formel I, können dabei durch geeignete Schutzgruppen maskiert werden. Geeignete Schutzgruppen für Aminofunktionen sind beispielweise die t-Butoxycarbonyl-, die Benzyloxycarbonyl-oder die Phtalolylgruppe sowie die Trityl- oder Tosylschutzgruppe. Geeignete Schutzgruppen für die Carboxylfunktion sind beispielweise Alkyl-, Aryl- oder Arylalkylester. Schutzgruppen können durch wohlbekannte oder hier beschriebene Techniken eingeführt und entfernt werden (siehe Green, T.W., Wutz, P.G.M., Protective Groups in Organic Synthesis (1991), 2nd Ed., Wiley-Interscience, oder Kocienski, P., Protecting Groups (1994), Thieme). Der Begriff Schutzgruppe kann auch entsprechende polymergebundene Schutzgruppen umfassen. Solcherart maskierte Verbindungen gemäß Formel (I), in der beispielsweise die funktionellen Gruppen der Reste U, V, X oder W gegebenenfalls ebenfalls maskiert sein können, können, obwohl gegebenenfalls selber pharmakologisch nicht aktiv, gegebenenfalls nach Administration in Säugern durch Metabolisierung zu den erfindungsgemäßen, pharmakologisch aktiven Verbindungen umgewandelt werden.

Die Erfindung betrifft ferner ein Verfahren gemäß Anspruch 3 zur Herstellung der Verbindung der Formel I und/oder einer stereoisomeren Form der Verbindung der Formel I und/oder eines physiologisch verträglichen Salzes der Verbindung der Formel I, wobei in Formel I jeweils n=0 ist. Die Verbindung der Formel I können z. B. nach Schema 1 herstellt, wobei R, U, V, W, X und Y die jeweils oben angegebenen Bedeutungen haben -und LG eine geeignete Abgangsgruppe wie -Cl, -Br, -I, -O-Tosyl oder -O-Mesyl darstellen:

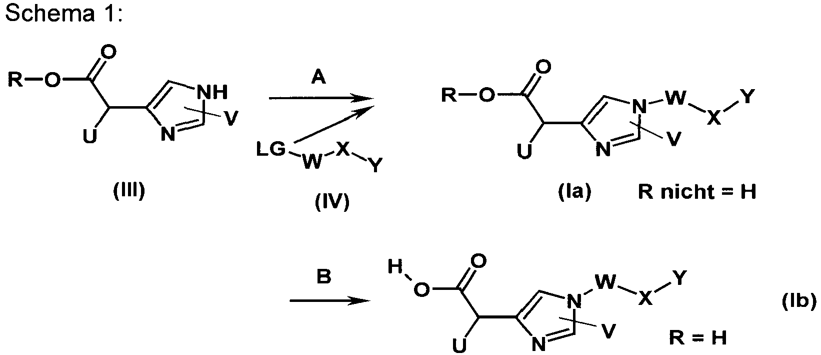

In einem Verfahrensschritt A wird die Verbindung der Formel III in einem polaren aprotischen Lösungsmittel wie Dimethylformamid (DMF) oder Tetrahydrofuran (THF) gelöst, mit einer geeigneten Base wie Natriumhydrid oder Lithiumhexamethyldi-silazan deprotoniert und mit Verbindungen der Formel IV zur Reaktion gebracht. Die erhaltenen Verbindungen la können in einem Verfahrensschritt B durch geeignete Spaltung der Estergruppe, wobei R nicht H ist (vgl. z. B. Kocienski, P.J., Protecting groups, Thieme 1994) zu den Verbindungen Ib mit R = H umgesetzt werden.

Ein alternatives Verfahren zur Herstellung der Verbindung der Formel I und/oder einer stereoisomeren Form der Verbindung der Formel I und/oder eines physiologisch verträglichen Salzes der Verbindung der Formel I, wobei in Formel I jeweils n=0 ist, das dadurch gekennzeichnet ist, dass man die Verbindung der Formel I nach Schema 3 herstellt, wobei PG, R, U, V, W, X und Y die jeweils oben angegebenen Bedeutungen haben und LG eine geeignete Abgangsgruppe wie -Cl, -Br, -I, -O-Tosyl oder -O-Mesyl darstellen:

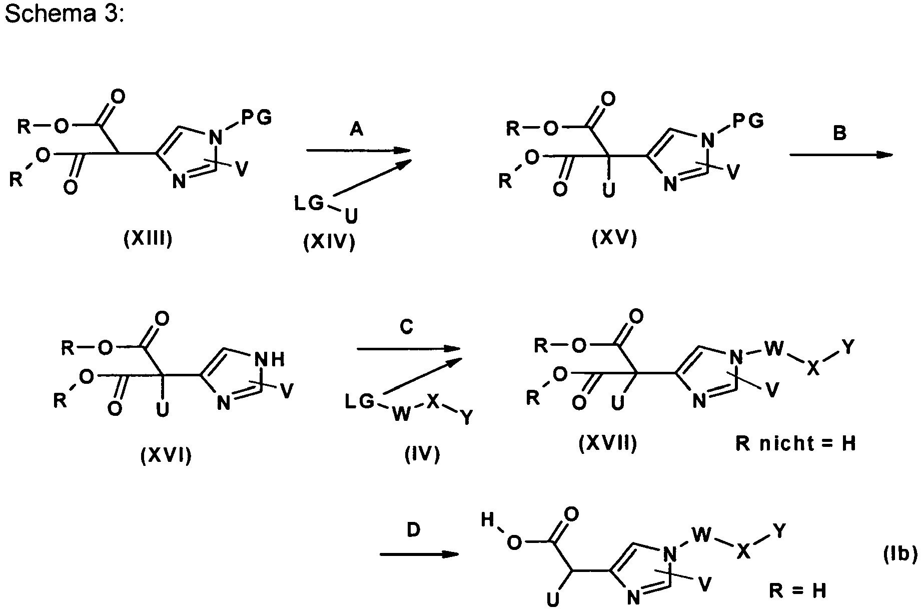

In einem Verfahrensschritt A wird die Verbindung der Formel XIII in einem polaren aprotischen Lösungsmittel wie Dimethylformamid (DMF) oder Tetrahydrofuran (THF) gelöst, mit einer geeigneten Base wie Cäsiumcarbonat, Natriumhydrid oder Lithiumhexamethyldi-silazan deprotoniert und mit Verbindungen der Formel XIV zur Reaktion gebracht. Der Verfahrensschritt B umfaßt die Abspaltung der Imidazolschutzgruppe PG in Formel XV nach literaturüblichen Verfahren zu Verbindungen der Formel XVI. In einem Verfahrensschritt C wird die Verbindung der Formel XVI in einem polaren aprotischen Lösungsmittel wie Dimethylformamid (DMF) oder Tetrahydrofuran (THF) gelöst, mit einer geeigneten Base wie Cäsiumcarbonat, Natriumhydrid oder Lithiumhexamethyldi-silazan deprotoniert und mit Verbindungen der Formel IV zur Reaktion gebracht. Der Verfahrensschritt D umfaßt die Esterspaltung und Decarboxylierung nach literaturüblichen Verfahren zum Beispiel durch Erhitzen in wässrigen Säuren oder Laugen.

Die Synthese von Verbindungen der Formel III kann in Anlehnung zu dem in J. Med. Chem. 2003, 46, 5294-5297 angegebenen Verfahren erfolgen. Die Herstellung der Verbindungen der Formel IV erfolgt nach bekannten Verfahren und kann beispielsweise in Anlehnung an die in Ewing, William R. et al. PCT Int. Appl. (2001), 460 pp. WO 0107436 A2 oder in Bioorg. Med. Chem. Lett. 2004, 14, 4191-4195 beschriebenen Methoden erfolgen. 2-Brommethyl-6-chlor-thieno[2,3-b]pyridin kann aus dem in Journal of the Chemical Society, Perkin Transactions 1: Organic and Bio-Organic Chemistry 1981, 9, 2509-17 beschriebenen 6-Chlor-thieno[2,3-b]pyridine-2-carbaldehyd nach obigen Verfahren gewonnen werden.

Die Umsetzungen können bei normalem, erhöhtem oder bei erniedrigtem Druck durchgeführt werden. Im Allgemeinen arbeitet man bei Normaldruck.

Als Lösungsmittel für die Verfahrensschritte (A) und (B) in Schema 1 sowie für die Verfahrensschritte (D) und (F) in Schema 2 und (A) und (C) in Schema 3 eignen sich inerte organische Lösungsmittel. Hierzu gehören beispielsweise Ether wie Dioxan, THF oder 1,2-Dimethoxyethan, Kohlenwasserstoffe wie Cyclohexan, Benzol, Toluol oder Xylol, Nitroaromaten wie Nitrobenzol, Carbonsäureamide wie Dimethylformamid oder Dimethylacetamid, Alkylsulfoxide wie Dimethylsulfoxid, aliphatische Nitrile wie Acetonitril, oder andere Lösungsmittel wie N-Methylpyrrolidinon. Bevorzugte Lösungsmittel für (B) in Schema 3 sind niedere aliphatische Alkohole wie Methanol oder Ethanol. Für den Schritt (G) und (D) in Schema 3 kommen wäßrige säurehaltige Lösungsmittelgemische in Frage. Ebenso ist es möglich, Gemische der jeweils genannten Lösungsmittel anzuwenden.

Als Basen für die Verfahrensschritte (A) und (B) in Schema 1 und (A) und (C) in Schema 3 eignen sich die üblichen anorganischen und organischen Basen. Hierzu gehören bevorzugt Alkali- und Erdalkalicarbonate wie Natrium-, Kalium oder Calciumcarbonat, Alkalihydride wie Natriumhydrid, Amide wie Lithium-bis(trimethylsilyl)amid oder Lithium-diisopropylamid, organische Amine wie Pyridin, 4-N,N-Dimethylaminopyridin, Triethylamin, Ethyldiisopropylamin, N-Methylmorpholin, N-Methylpiperidin, 1,5-Diazabicyclo(4.3.0)non-5-en (DBN) oder 1,8-Diazabicyclo(5.4.0)undec-7-en (DBU), oder metallorganische Verbindungen wie Butyllithium oder Phenyllithium. Besonders bevorzugt sind Natriumhydrid, Lithium-bis(trimethylsilyl)amid und Triethylamin.

Eine nach Schema 1 hergestellte Verbindung der Formel I, oder eine geeignete Vorstufe der Formel I, die aufgrund ihrer chemischen Struktur in enantiomeren Formen auftritt, kann durch Salzbildung mit enantiomerenreinen Säuren oder Basen, Chromatographie an chiralen Stationärphasen oder Derivatisierung mittels chiraler enantiomerenreinen Verbindungen wie Aminosäuren, Trennung der somit erhaltenen Diastereomeren, und Abspaltung der chiralen Hilfsgruppen in die reinen Enantiomeren aufgetrennt werden (Verfahren b), oder die nach Schema 1 hergestellte Verbindung der Formel I kann entweder in freier Form isoliert oder im Falle des Vorliegens von sauren oder basischen Gruppen in physiologisch verträgliche Salze umwandelt werden (Verfahren d).

Im Verfahrensschritt c) wird die Verbindung der Formel I, sofern sie als Gemisch von Diastereomeren oder Enantiomeren auftritt oder bei der gewählten Synthese als deren Gemische anfällt, in die reinen Stereoisomeren getrennt, entweder durch Chromatographie an einem gegebenenfalls chiralen Trägermaterial, oder, sofern die racemische Verbindung der Formel I zur Salzbildung befähigt ist, durch fraktionierte Kristallisation der mit einer optisch aktiven Base oder Säure als Hilfsstoff gebildeten diastereomeren Salze. Als chirale Stationärphasen für die dünnschicht- oder säulenchromatographische Trennung von Enantiomeren eignen sich zum Beispiel modifizierte Kieselgelträger (sogenannte Pirkle-Phasen) sowie hochmolekulare Kohlenhydrate wie Triacetylcellulose. Für analytische Zwecke sind nach entsprechender, dem Fachmann bekannter Derivatisierung, auch gaschromatographische Methoden an chiralen Stationärphasen anwendbar. Zur Enantiomerentrennung der racemischen Carbonsäuren werden mit einer optisch aktiven, in der Regel kommerziell erhältlichen Base wie (-)-Nicotin, (+)- und (-)-Phenylethylamin, Chininbasen, L-Lysin oder L-und D-Arginin die unterschiedlich löslichen diastereomeren Salze gebildet, die schwerer lösliche Komponente als Feststoff isoliert, das leichter lösliche Diastereomer aus der Mutterlauge abgeschieden und aus den so gewonnenen diastereomeren Salzen die reinen Enantiomeren gewonnen. Auf prinzipiell gleiche Weise kann man die racemischen Verbindungen der Formel I, die eine basische Gruppe wie eine Aminogruppe enthalten, mit optisch aktiven Säuren, wie (+)-Campher-10-sulfonsäure, D- und L- Weinsäure, D-und L-Milchsäure sowie (+) und (-)-Mandelsäure in die reinen Enantiomeren überführen. Auch kann man chirale Verbindungen, die Alkohol- oder Amin-funktionen enthalten, mit entsprechend aktivierten oder gegebenenfalls N-geschützten enantiomerenreinen Aminosäuren in die entsprechenden Ester oder Amide, oder umgekehrt chirale Carbonsäuren mit carboxygeschützten enantiomerenreinen Aminosäuren in die Amide oder mit enantiomerenreinen Hydroxycarbonsäuren wie Milchsäure, in die entsprechenden chiralen Ester überführen. Sodann kann die Chiralität des in enantiomerenreiner Form eingebrachten Aminosäure- oder Alkoholrestes zur Trennung der Isomeren genutzt werden, indem man eine Trennung der nunmehr vorliegenden Diastereomeren durch Kristallisation oder Chromatographie an geeigneten Stationärphasen vornimmt und danach den mitgeführte chiralen Molekülteil mittels geeigneter Methoden wieder abspaltet.

Weiterhin ergibt sich bei einigen der erfindungsgemäßen Verbindungen die Möglichkeit, zur Herstellung der Gerüststrukturen diastereo- oder enantiomerenreine Ausgangsprodukte einzusetzen. Dadurch können auch andere oder vereinfachte Verfahren zur Aufreinigung der Endprodukte eingesetzt werden. Diese Ausgangsprodukte wurden zuvor nach literatur-bekannten Verfahren enantiomeren-oder diastereomerenrein hergestellt. Das kann insbesondere bedeuten, dass in der Synthese der Grundgerüste entweder enantioselektive Verfahren zum Einsatz kommen, oder aber eine Enantiomeren- (oder Diastereomeren-) Trennung auf früher Synthesestufe und nicht erst auf der Stufe der Endprodukte durchgeführt wird. Ebenso kann eine Vereinfachung der Trennungen dadurch erreicht werden, dass zwei- oder mehrstufig vorgegangen wird.

Saure oder basische Produkte der Verbindung der Formel I können in Form ihrer Salze oder in freier Form vorliegen. Bevorzugt sind pharmakologisch verträgliche Salze, beispielsweise Alkali- oder Erdalkalimetallsalze wie Hydrochloride, Hydrobromide, Sulfate, Hemisulfate, alle möglichen Phosphate sowie Salze der Aminosäuren, natürlicher Basen oder Carbonsäuren. Die Herstellung physiologisch verträglicher Salze aus zur Salzbildung befähigten Verbindungen der Formel I, einschließlich derer stereoisomerer Form, gemäß Verfahrensschritt c) erfolgt in an sich bekannter Weise. Die Verbindungen der Formel I bilden mit basischen Reagenzien wie Hydroxiden, Carbonaten, Hydrogencarbonaten, Alkoholaten sowie Ammoniak oder organischen Basen, beispielsweise Trimethyl- oder Triethylamin, Ethanolamin, Diethanolamin oder Triethanolamin, Trometamol oder auch basischen Aminosäuren, etwa Lysin, Ornithin oder Arginin, stabile Alkali-, Erdalkali- oder gegebenenfalls substituierte Ammoniumsalze. Sofern die Verbindungen der Formel I basische Gruppen aufweisen, lassen sich mit starken Säuren auch stabile Säureadditionssalze herstellen. Hierfür kommen sowohl anorganische als auch organische Säuren wie Chlorwasserstoff-, Bromwasserstoff-, Schwefel-, Hemischwefel-, Phosphor-, Methansulfon-, Benzolsulfon-, p-Toluolsulfon-, 4-Brombenzol-sulfon-, Cyclohexylamidosulfon-, Trifluormethylsulfon-, 2-Hydroxyethansulfon-, Essig-, Oxal-, Wein-, Bernstein-, Glycerolphosphor-, Milch-, Äpfel-, Adipin-, Citronen-, Fumar-, Malein-, Glucon-, Glucuron- Palmitin-, oder Trifluoressigsäure in Frage.

Die Erfindung betrifft auch Arzneimittel, gekennzeichnet durch einen wirksamen Gehalt an mindestens einer Verbindung der Formel I und/oder eines physiologisch verträglichen Salzes der Verbindung der Formel I und/oder eine gegebenenfalls stereoisomere Form der Verbindung der Formel I, zusammen mit einem pharmazeutisch geeigneten und physiologisch verträglichen Trägerstoff, Zusatzstoff und/oder anderen Wirk- und Hilfsstoffen.

Aufgrund der pharmakologischen Eigenschaften eignen sich die erfindungsgemäßen Verbindungen zur Prophylaxe, Sekundär-Prevention und Therapie all solcher Erkrankungen, die durch eine Hemmung von TAFIa behandelbar sind. So eignen sich TAFIa Inhibitoren sowohl für eine prophylaktische als auch für eine therapeutische Anwendung am Menschen. Sie eignen sich sowohl für eine akute Behandlung als auch für eine Langzeittherapie. TAFIa Inhibitoren können eingesetzt werden in Patienten, die an Störungen des Wohlbefindens oder Krankheiten leiden, die mit Thrombosen, Embolien, Hyperkoagulabilität oder fibrotischen Veränderungen einhergehen.

Dazu gehören der Myokardinfarkt, die Angina pectoris und alle anderen Formen des akuten Koronarsyndroms, der Schlaganfall, die peripher vaskulären Erkrankungen, die tiefe Venenthrombose, die Lungenembolie, embolische oder thrombotische Ereignisse bedingt durch kardiale Arrhythmien, kardiovaskuläre Ereignisse wie Restenose nach Revaskularisierung, Angioplastie und ähnlichen Eingriffen wie Stentimplantationen und Bypass-Operationen. Weiterhin können TAFIa Inhibitoren eingesetzt werden bei allen Eingriffen, die zu einem Kontakt des Blutes mit Fremdoberflächen führen beispielsweise bei Dialysepatienten und Patienten mit Verweilkathetern. TAFIa Inhibitoren können eingesetzt werden, um die Thrombosegefahr nach chirurgischen Eingriffen wie bei Knie- und Hüftgelenksoperationen zu reduzieren.

TAFIa Inhibitoren eignen sich für die Behandlung von Patienten mit disseminierter intravaskulärer Koagulation, Sepsis und anderen intravaskulären Ereignissen, die mit einer Entzündung einhergehen. Weiterhin eignen sich TAFIa Inhibitoren für die Prophylaxe und Behandlung von Patienten mit Atherosklerose, Diabetes und dem metabolischen Syndrom und deren Folgen. Störungen des hämostatischen Systems (beispielsweise Fibrinablagerungen) wurden impliziert in Mechanismen, die zu Tumorwachstum und Tumormetastasierung führen, sowie bei entzündlichen und degenerativen Gelenkserkrankungen wie der rheumatoiden Arthritis und der Arthrose. TAFIa Inhibitoren eignen sich zur Verlangsamung oder Verhinderung solcher Prozesse.

Weitere Indikationen für den Einsatz von TAFIa Inhibitoren sind fibrotische Veränderungen der Lunge wie die chronische obstruktive Lungenerkrankung, das adult respiratory distress syndrome (ARDS) und des Auges wie Fibrinablagerungen nach Augenoperationen. TAFIa Inhibitoren eignen sich auch zur Verhinderung und/oder Behandlung von Narbenbildung.

Die Applikation der erfindungsgemäßen Arzneimittel kann durch orale, inhalative, rektale oder transdermale Applikation oder durch subkutane, intraartikuläre, intraperitoneale oder intravenöse Injektion erfolgen. Bevorzugt ist die orale Applikation. Eine Beschichtung mit TAFIa Inhibitoren von Stents und anderen Oberflächen, die im Körper mit Blut in Kontakt kommen, ist möglich.

Die Erfindung betrifft auch ein Verfahren zur Herstellung eines Arzneimittels, das dadurch gekennzeichnet, dass man mindestens eine Verbindung der Formel I mit einem pharmazeutisch geeigneten und physiologisch verträglichen Träger und gegebenenfalls weiteren geeigneten Wirk-, Zusatz- oder Hilfsstoffen in eine geeignete Darreichungsform bringt.

Geeignete feste oder galenische Zubereitungsformen sind beispielsweise Granulate, Pulver, Dragees, Tabletten, (Mikro)Kapseln, Suppositorien, Sirupe, Säfte, Suspensionen, Emulsionen, Tropfen oder injizierbare Lösungen sowie Präparate mit protrahierter Wirkstoff-Freigabe, bei deren Herstellung übliche Hilfsmittel wie Trägerstoffe, Spreng-, Binde-, Überzugs-, Quellungs-, Gleit- oder Schmiermittel, Geschmacksstoffe, Süßungsmittel und Lösungsvermittler Verwendung finden. Als häufig verwendete Hilfsstoffe seien Magnesiumcarbonat, Titandioxid, Laktose, Mannit und andere Zucker, Talkum, Milcheiweiß, Gelatine, Stärke, Cellulose und ihre Derivate, tierische und pflanzliche Öle wie Lebertran, Sonnenblumen-, Erdnuss- oder Sesamöl, Polyethylenglykol und Lösungsmittel wie etwa steriles Wasser und ein- oder mehrwertige Alkohole wie Glycerin, genannt.

Vorzugsweise werden die pharmazeutischen Präparate in Dosierungseinheiten hergestellt und verabreicht, wobei jede Einheit als aktiven Bestandteil eine bestimmte Dosis der erfindungsgemäßen Verbindung der Formel I enthält. Bei festen Dosierungseinheiten wie Tabletten, Kapseln, Dragees oder Suppositorien, kann diese Dosis bis zu etwa 1000 mg, bevorzugt jedoch etwa 50 bis 300 mg und bei Injektionslösungen in Ampullenform bis zu etwa 300 mg, vorzugsweise aber etwa 10 bis 100 mg, betragen.

Für die Behandlung eines erwachsenen, etwa 70 kg schweren Patienten sind je nach Wirksamkeit der Verbindung gemäß Formel I, Tagesdosen von etwa 2 mg bis 1000 mg Wirkstoff, bevorzugt etwa 50 mg bis 500 mg indiziert. Unter Umständen können jedoch auch höhere oder niedrigere Tagesdosen angebracht sein. Die Verabreichung der Tagesdosis kann sowohl durch Einmalgabe in Form einer einzelnen Dosierungseinheit oder aber mehrerer kleinerer Dosierungseinheiten als auch durch Mehrfachgabe unterteilter Dosen in bestimmten Intervallen erfolgen.

TAFIa Inhibitoren können sowohl als Monotherapie als auch in Kombination oder gemeinsam mit allen Antithrombotika (Antikoagulanzien und Plättchenaggregationshemmer), Thrombolytika (Plasminogenaktivatoren jeglicher Art), anderen profibrinolytisch wirksamen Substanzen, Blutdrucksenkern, Regulatoren des Blutzuckers, Lipidsenkern und Antiarrhythmika verabreicht werden.

### Beispiele

Endprodukte werden in der Regel durch massenspektroskopische Methoden (FAB-, ESI-MS) und ¹H-NMR bestimmt, angegeben sind jeweils der Hauptpeak oder die beiden Hauptpeaks. Temperaturangaben in Grad Celsius, Ausb. bedeutet Ausbeute, RT oder Raumtemp. bedeutet Raumtemperatur (21°C bis 24 °C), i. Vak. steht für im Vakuum. Verwendete Abkürzungen sind entweder erläutert oder entsprechen den üblichen Konventionen.

Wenn nicht anders aufgeführt, wurden die LC/MS-Analysen unter folgenden Bedingungen durchgeführt:
Methode A: Säule: YMC Jsphere 33x2,1 mm, Packungsmaterial 4 µm, Laufmittel: CH₃CN + 0,05% Trifluoressigsäure (TFA): H₂O + 0,05% TFA, Gradient: 5:95 (0 min) nach 95:5 (2,5 min) nach 95:5 (3,0 min), Fluß: 1,3 mL/min., Temperatur: 30 °C.
Methode B: Säule: YMC Jsphere 33x2,1 mm, Packungsmaterial 4 µm, Laufmittel: CH₃CN + 0,05% TFA: H₂O + 0,05% TFA, Gradient: 5:95 (0 min) nach 95:5 (3,4 min) nach (4,4 min), Fluss: 1 mL/min, Temperatur: 30 °C.

Soweit nicht anders angegeben, wurden chromatographische Trennungen an Kieselgel mit Ethylacetat/Heptan-Gemischen als Laufmittel durchgeführt. Präparative Trennungen an Reversed Phase-(RP)-Kieselgel (HPLC) wurden, soweit nicht anders angegeben, unter folgenden Bedingungen durchgeführt: Säule Merck Hibar RT 250-25 LiChrospher 100 RP-18e 5µm, mobile Phase A: H₂O + 0,1% TFA, Phase B: 80% Acetonitril + 0,1% TFA, Fluss 25 ml/min, 0-7 min 100% A , 7-22 min auf 100% B, 22-30 min 100% B, 30-33 min auf 100% A, 33-35 min 100% A.

Das Abdampfen von Lösungsmitteln geschah in der Regel unter vermindertem Druck bei 35 °C bis 45 °C am Rotationsverdampfer.

### Beispiel 1

### 3-(6-Amino-pyridin-3-yl)-2-{1-[5-(5-chloro-thiophen-2-yl)-isoxazol-3-ylmethyl]-1H-imidazol-4-yl}-propionsäure

### a) 3-(6-Amino-pyridin-3-yl)-2-{1-[5-(5-chloro-thiophen-2-yl)-isoxazol-3-ylmethyl]-1H-imidazol-4-yl}-propionsäuremethylester Trifluoracetat

Eine Lösung von 0,300 g (1,218 mmol) 3-(6-Amino-pyridin-3-yl)-2-(1H-imidazol-4-yl)-propionsäuremethylester in 5 mL absolutem Dimethylformamid (DMF) wurde unter Argon mit 0,032 g (1,340 mmol) Natriumhydrid versetzt und 30 min bei RT gerührt. Anschließend fügte man 0,339 g (1,218 mmol) 3-Brommethyl-5-(5-chloro-thiophen-2-yl)-isoxazol hinzu, rührte für weiter 3 h bei RT und engte das Reaktionsgemisch ein. Der Rückstand wurde über präparative HPLC gereinigt. Gefriertrocknung der Wertfraktionen lieferte die Titelverbindung als Trifluoressigsäuresalz. Ausb. 0,450 g (66%).

LC/MS: Rₜ = 1,17 min, [M+H]⁺ = 444 (Methode A).

### b) 3-(6-Amino-pyridin-3-yl)-2-{1-[5-(5-chloro-thiophen-2-yl)-isoxazol-3-ylmethyl]-1H-imidazol-4-yl}-propionsäure

Eine Lösung bestehend aus 0,45 g (0,81 mmol) 3-(6-Amino-pyridin-3-yl)-2-{1-[5-(5-chloro-thiophen-2-yl)-isoxazol-3-ylmethyl]-1H-imidazol-4-yl}-propionsäuremethylester in 10 mL halbkonzentrierter Salzsäure wurde 1 h bei 90 °C gerührt. Nach dem Abkühlen wurde eingeengt und das Rohprodukt wurde über präparative HPLC gereinigt. Gefriertrocknung der Wertfraktionen lieferte die Titelverbindung als Trifluoressigsäuresalz. Ausbeute 0,35 g (80%).

LC/MS: Rₜ = 1,17 min, [M+H]⁺ = 430, Chloropattern (Methode A). ¹H-NMR (DMSO-d₆, 500 MHz): δ [ppm] = 13,75 (s, br, 1 H), 8,81 (s, br, 1 H), 8,00 (s, br, 2H), 7,72 (m, 2H), 7,61 (d, 1H), 7,50 (s, 1 H), 7,32 (d, 1 H), 6,93 (s, 1 H), 6,88 (d, 1 H), 5,51 (s, 1 H), 4,05 (t, 1 H), 3,14 (dd, 1 H), 3,00 (dd, 1 H).

### Beispiel 2

### 3-(6-Amino-pyridin-3-yl)-2-[1-(5-phenyl-isoxazol-3-ylmethyl)-1 H-imidazol-4-yl]-propionsäure

### a) 3-(6-Amino-pyridin-3-yl)-2-[1-(5-phenyl-isoxazol-3-ylmethyl)-1 H-imidazol-4-yl]-propionsäu remethylester

Eine Lösung von 0,400 g (1,624 mmol) 3-(6-Amino-pyridin-3-yl)-2-(1 H-imidazol-4-yl)-propionsäuremethylester in 15 mL absolutem DMF wurde unter Argon mit 0,041 g (1,624 mmol) Natriumhydrid versetzt und 30 min bei RT gerührt. Anschließend fügte man 0,387 g (1,624 mmol) 3-Brommethyl-5-phenyl-isoxazol hinzu, rührte für weiter 3h bei RT und engte dann das Reaktionsgemisch ein. Der Rückstand wurde in Ethylacetat aufgenommen, es wurde mit Wasser gewaschen, über Na₂SO₄ getrocknet und eingeengt. Das Rohprodukt wurde an Kieselgel chromatographiert (Laufmittel Dichlormethan/Methanol/Essigsäure/Wasser 90:10:1:1). Gefriertrocknen der Wertfraktionen lieferte die Titelverbindung als Acetat. Ausb. 0,600 g (80%).

LC/MS: Rₜ = 1,1 min, [M+H]⁺ = 404 (Methode B).

### b) 3-(6-Amino-pyridin-3-yl)-2-[1-(5-phenyl-isoxazol-3-ylmethyl)-1 H-imidazol-4-yl]-propionsäure

Eine Lösung von 0,564 g (1,216 mmol) der Verbindung aus Beispiel 2a) in 45 mL THF/Methanol (2:1, v/v) wurde mit 6,1 mL (6,1 mmol) 1M Lithiumhydroxyd-Lösung versetzt und 2h bei 50 °C erwärmt. Die organischen Lösungsmittel wurden abdestilliert und der Rückstand über einer präparativen HPLC gereinigt. Gefriertrocknung der Wertfraktionen lieferte die Titelverbindung als Trifluoressigsäuresalz. Ausbeute 0,314 g (51%).

LC/MS: Rₜ = 1,08 min, [M+H]⁺ = 390 (Methode A). ¹H-NMR (DMSO-d₆, 500 MHz): δ

[ppm] = 13,7 (s, br, 1 H), 8,89 (s, br, 1 H), 8,03 (s, br, 2H), 7,87 (d, 2H), 7,72 (m, 2H), 7,55 (m, 4H), 7,03 (s, 1 H), 6,88 (d, 1 H), 5,53 (s, 2H), 4,09 (t, 1 H), 3,17 (dd, 1 H), 3,01 (dd, 1 H).

### Beispiel 3 (nicht erfindungsgemäß)

### 3-(6-Amino-pyridin-3-yl)-2-{1-[2-(5-chloro-thiophen-2-yl)-thiazol-5-ylmethyl]-1H-imidazol-4-yl}-propionsäure

Die Titelverbindung wurde analog zu Beispiel 2 als Trifluoressigsäuresalz aus 5-Bromomethyl-2-(5-chlor-thiophen-2-yl)-thiazol gewonnen.

LC/MS: Rₜ = 1,03 min, [M+H]⁺ = 446, Chloropattern (Methode A). ¹H-NMR (DMSO-d₆, 500 MHz): ö [ppm] = 13,8 (s, br, 1 H), 8,65 (s, br, 1 H), 8,02 (s, br, 2H), 7,98 (s, 1 H), 7,72 (m, 2H), 7,58 (d, 1 H), 7,45 (s, 1 H), 7,21 (d, 1H), 6,85 (d, 1H), 5,61 (s, 2H), 4,03 (t, 1H), 3,12 (dd, 1 H), 2,99 (dd, 1 H).

### Beispiel 4 (nicht erfindungsgemäß)

### 3-(6-Amino-pyridin-3-yl)-2-[1-(6-chlor-thieno[2,3-b]pyridin-2-ylmethyl)-1 H-imidazol-4-yl]-propionsäure

Die Titelverbindung wurde analog zu Beispiel 2 als Trifluoressigsäuresalz aus 2-Brommethyl-6-chlor-thieno[2,3-b]pyridin gewonnen.

LC/MS: Rₜ = 0,85 min, [M+H]⁺ = 414, Chloropattern (Methode A). ¹H-NMR (DMSO-d₆, 500 MHz): δ [ppm] = 13,8 (s, br, 1 H), 8,65 (s, br, 1 H), 8,32 (d, 1H), 7,94 (s, br, 2H), 7,68 (m, 2H), 7,56 (d, 1 H), 7,46 (m, 2H), 6,83 (d, 1 H), 5,65 (s, 2H), 4,02 (t, 1 H), 3,12 (dd, 1 H), 2,99 (dd, 1 H).

### Beispiel 5

### 3-(6-Amino-pyridin-3-yl)-2-{1-[5-(4-methoxy-phenyl)-isoxazol-3-ylmethyl]-1H-imidazol-4-yl}-propionsäure

Die Titelverbindung wurde analog zu Beispiel 2 als Trifluoressigsäuresalz aus 3-Brommethyl-5-(4-methoxy-phenyl)-isoxazol gewonnen.

LC/MS: Rₜ = 1,04 min, [M+H]⁺ = 420 (Methode A). ¹H-NMR (DMSO-d₆, 500 MHz): ö [ppm] = 13,8 (s, br, 1 H), 8,91 (s, br, 1 H), 8,03 (s, br, 2H), 7,78 (d, 2H), 7,72 (m, 2H), 7,57 (s, 1H), 7,08 (d, 2H), 6,91 (s, 1 H), 6,88 (d, 1 H), 5,52 (s, 2H), 4,12 (t, 1 H), 3,85 (s, 3H), 3,18 (dd, 1H), 3,02 (dd, 1 H).

### Beispiel 6 (nicht erfindungsgemäß)

### 3-(6-Amino-pyridin-3-yl)-2-{1-[5-(3-methyl-butyl)-isoxazol-3-ylmethyl]-1H-imidazol-4-yl}-propionsäure

Die Titelverbindung wurde analog zu Beispiel 2 als Trifluoressigsäuresalz aus 3-Brommethyl-5-(3-methyl-butyl)-isoxazol gewonnen.

LC/MS: Rₜ = 1,11 min, [M+H]⁺ 384 = (Methode A). ¹H-NMR (DMSO-d₆, 500 MHz): δ [ppm] = 13,7 (s, br, 1 H), 8,82 (s, br, 1 H), 8,02 (s, br, 2H), 7,72 (m, 2H), 7,49 (s, 1 H), 6,87 (d, 1H), 6,30 (s, 1H), 5,45 (s, 2H), 4,08 (t, 1H), 3,16 (dd, 1H), 3,02 (dd, 1H), 2,76 (t, 2H), 1,59-1,48 (m, 3H), 0,88 (d, 6H).

### Beispiel 7

### 3-(6-Amino-pyridin-3-yl)-2-{1-[5-(5-chlor-thiophen-2-yl)-[1,3,4]thiadiazol-2-ylmethyl]-1H-imidazol-4-yl}-propionsäure

Die Titelverbindung wurde analog zu Beispiel 2 als Trifluoressigsäuresalz aus 2-Brommethyl-5-(5-chlor-thiophen-2-yl)-[1,3,4]thiadiazol gewonnen.

LC/MS: Rₜ = 1,09 min, [M+H]⁺ = 447, Chloropattern (Methode A). ¹H-NMR (DMSO-d₆, 500 MHz): δ [ppm] = 13,8 (s, br, 1H), 8,68 (s, br, 1 H), 7,98 (s, br, 2H), 7,73 (m, 3H), 7,47 (s, 1 H), 7,32 (d, 1 H), 6,87 (d, 1 H), 5,87 (s, 2H), 4,05 (t, 1 H), 3,13 (dd, 1 H), 3,02 (dd, 1 H).

### Beispiel 8

### 3-(6-Amino-pyridin-3-yl)-2-{1-[5-(4-chlor-phenyl)-[1,3,4]thiadiazol-2-ylmethyl]-1H-imidazol-4-yl}-propionsäure

Die Titelverbindung wurde analog zu Beispiel 2 als Trifluoressigsäuresalz aus 2-Chlormethyl-5-(4-chlor-phenyl)-[1,3,4]thiadiazol gewonnen.

LC/MS: Rₜ = 1,13 min, [M+H]⁺ = 441, Chloropattern (Methode A). ¹H-NMR (DMSO-d₆, 500 MHz): δ [ppm] = 13,8 (s, br, 1H), 8,70 (s, br, 1 H), 7,98 (m, 4H), 7,71 (d, 2H), 7,62 (d, 2H), 7,52 (s, 1 H), 6,88 (d, 1 H), 5,90 (s, 2H), 4,03 (t, 1 H), 3,15 (dd, 1 H), 3,02 (dd, 1 H).

### Beispiel 9 (nicht erfindungsgemäß)

### 3-(6-Amino-pyridin-3-yl)-2-[1-(5-tert-butyl-[1,3,4]thiadiazol-2-ylmethyl)-1H-imidazol-4-yl]-propionsäure

Die Titelverbindung wurde analog zu Beispiel 2 als Trifluoressigsäuresalz aus 2-tert-Butyl-5-chlormethyl-[1,3,4]thiadiazol gewonnen.

LC/MS: Rₜ = 0,89 min, [M+H]⁺ = 387 (Methode A). ¹H-NMR (DMSO-d₆, 500 MHz): δ [ppm] = 13,6 (s, br, 1H), 8,55 (s, br, 1H), 8,01 (s, br ,2H), 7,72 (m, 2H), 7,46 (s, 1 H), 6,86 (d, 1 H), 5,79 (s, 2H), 4,03 (t, 1 H), 3,12 (dd, 1 H), 3,00 (dd, 1 H), 1,42 (s, 9H).

### Beispiel 10

### 3-(6-Amino-pyridin-3-yl)-2-{1-[5-(4-trifluormethyl-phenyl)-[1,2,4]oxadiazol-3-ylmethyl]-1H-imidazol-4-yl}-propionsäure

Die Titelverbindung wurde analog zu Beispiel 2 als Trifluoressigsäuresalz aus 3-Chlormethyl-5-(4-trifluormethyl-phenyl)-[1,2,4]oxadiazol gewonnen.

LC/MS: Rₜ = 1,12 min, [M+H]⁺ = 459 (Methode A). ¹H-NMR (DMSO-d₆, 500 MHz): δ [ppm] = 13,8 (s, br, 1 H), 8,72 (s, br, 1 H), 8,30 (d, 2H), 8,05 (d, 2H), 8,03 (s, 2H), 7,73 (m, 2H), 7,52 (s, 1 H), 6,87 (d, 1 H), 5,72 (s, 2H), 4,08 (t, 1 H), 3,15 (dd, 1 H), 3,02 (dd, 1 H).

### Beispiel 11

### 3-(6-Amino-pyridin-3-yl)-2-{1-[5-(3-trifluormethyl-phenyl)-[1,2,4]oxadiazol-3-ylmethyl]-1H-imidazol-4-yl}-propionsäure

Die Titelverbindung wurde analog zu Beispiel 2 als Trifluoressigsäuresalz aus 3-Chlormethyl-5-(3-trifluormethyl-phenyl)-[1,2,4]oxadiazol gewonnen.

LC/MS: Rₜ = 1,07 min, [M+H]⁺ = 459 (Methode A). ¹H-NMR (DMSO-d₆, 500 MHz): δ [ppm] = 13,9 (s, br, 1 H), 8,79 (s, br, 1 H), 8,40 (d, 1 H), 8,35 (s, 1 H), 8,12 (d, 1 H), 8,02 (s, br, 2H), 7,92 (t, 1 H), 7,73 (m, 2H), 7,55 (s, 1 H), 6,88 (d, 1 H), 5,73 (s, 2H), 4,10 (t, 1 H), 3,17 (dd, 1H), 3,04 (dd, 1H).

### Beispiel 12

### 3-(6-Amino-pyridin-3-yl)-2-[1-(5-cyclopropyl-[1,3,4]thiadiazol-2-ylmethyl)-1H-imidazol-4-yl]-propionsäure

Die Titelverbindung wurde analog zu Beispiel 2 als Trifluoressigsäuresalz aus 2-Chlormethyl-5-cyclopropyl-[1,3,4]-thiadiazol gewonnen.

LC/MS: Rₜ = 0,62 min, [M+H]⁺ = 371 (Methode A). ¹H-NMR (DMSO-d₆, 500 MHz): δ [ppm] = 13,8 (s, br, 1H), 8,59 (s, br, 1H), 8,00 (s, br, 2H), 7,72 (m, 2H), 7,40 (s, 1H), 6,88 (d, 1 H), 5,75 (s, 2H), 4,03 (t, 1 H), 3,11 (dd, 1 H), 3,00 (dd, 1 H), 2,55 (m, 1 H), 1,21 (m, 2H), 0,99 (m, 2H).

### Beispiel 13

### 3-(6-Amino-pyridin-3-yl)-2-[1-(5-phenyl-[1,2,4]oxadiazol-3-ylmethyl)-1H-imidazol-4-yl]-propionsäure

Die Titelverbindung wurde analog zu Beispiel 2 als Trifluoressigsäuresalz aus 3-Chlormethyl-5-phenyl-[1,2,4]oxadiazol gewonnen.

LC/MS: Rₜ = 0,87 min, [M+H]⁺ = 391 (Methode A). ¹H-NMR (DMSO-d₆, 500 MHz): δ [ppm] = 13,7 (s, br, 1 H), 8,76 (s, br, 1 H), 8,10 (d, 2H), 8,06 (s, 2H), 7,73 (m, 3H), 7,68 (t, 2H), 7,52 (s, 1 H), 6,89 (d, 1H), 5,70 (s, 2H), 4,10 (t, 1 H), 3,16 (dd, 1H), 3,03 (dd, 1H).

### Beispiel 14

### 3-(6-Amino-pyridin-3-yl)-2-{1-[3-(3,4-dimethoxy-phenyl)-[1,2,4]oxadiazol-5-ylmethyl]-1H-imidazol-4-yl}-propionsäure

Die Titelverbindung wurde analog zu Beispiel 2 als Trifluoressigsäuresalz aus 5-Chlormethyl-3-(3,4-dimethoxy-phenyl)-[1,2,4]oxadiazol gewonnen.

LC/MS: Rₜ = 0,89 min, [M+H]⁺ = 451 (Methode A). ¹H-NMR (DMSO-d₆, 500 MHz): δ [ppm] = 13,5 (s, br, 1H), 8,62 (s, br, 1H), 7,99 (s, br, 2H), 7,71 (m, 2H), 7,53 (m, 2H), 7,42 (s, 1 H), 7,12 (d, 1 H), 6,88 (d, 1 H), 5,85 (s, 2H), 4,08 (t, 1 H), 3,72, (s, 3H), 3,71 (s, 3H), 3,15 (dd, 1 H), 3,03 (dd, 1 H).

### Beispiel 15

### 3-(6-Amino-pyridin-3-yl)-2-{1-[3-(4-methoxy-phenyl)-[1,2,4]oxadiazol-5-ylmethyl]-1H-imidazol-4-yl}-propionsäure

Die Titelverbindung wurde analog zu Beispiel 2 als Trifluoressigsäuresalz aus 3-Chlormethyl-5-(4-methoxy-phenyl)-[1,2,4]oxadiazol gewonnen.

LC/MS: Rₜ = 0,96 min, [M+H]⁺ = 421 (Methode A). ¹H-NMR (DMSO-d₆, 500 MHz): δ [ppm] = 13,7 (s, br, 1 H), 8,69 (s, br, 1 H), 8,03 (m, 4H), 7,73 (m, 2H), 7,50 (s, 1 H), 7,18 (d, 2H), 6,88 (d, 1H), 5,62 (s, 2H), 4,08 (t, 1H), 3,89 (s, 3H), 3,15 (dd, 1H), 3,01 (dd, 1H).

### Beispiel 16

### 3-(6-Amino-pyridin-3-yl)-2-{1-[3-(4'-isopropyl-phenyl)-[1,2,4]oxadiazol-5-ylmethyl]-1H-imidazol-4-yl}-propionsäure

Die Titelverbindung wurde analog zu Beispiel 2 als Trifluoressigsäuresalz aus 5-Chlormethyl-3-(4-isopropyl-phenyl)-[1,2,4]oxadiazol gewonnen.

LC/MS: Rₜ = 1,19 min, [M+H]⁺ = 433 (Methode A). ¹H-NMR (DMSO-d₆, 500 MHz): δ [ppm] = 13,5 (s, br, 1H), 8,53 (s, br, 1 H), 8,02 (s, br, 2H), 7,90 (d, 2H), 7,75 (m, 2H), 7,50 (s, 1 H), 7,46 (d, 2H), 6,88 (d, 1 H), 5,87 (s, 2H), 4,06 (t, 1 H), 3,15 (dd, 1 H), 3,04-2,92 (m, 2H), 1,22 (d, 6H).

### Beispiel 17

### 3-(6-Amino-pyridin-3-yl)-2-{1-[3-(4'-tert-butyl-phenyl)-[1,2,4]oxadiazol-5-ylmethyl]-1H-imidazol-4-yl}-propionsäure

Die Titelverbindung wurde analog zu Beispiel 2 als Trifluoressigsäuresalz aus 5-Chlormethyl-3-(4-tert-butyl-phenyl)-[1,2,4]oxadiazol gewonnen.

LC/MS: Rₜ = 1,26 min, [M+H]⁺ = 447 (Methode A). ¹H-NMR (DMSO-d₆, 500 MHz): δ [ppm] = 13,6 (s, br, 1 H), 8,52 (s, br, 1 H), 8,00 (s, br, 2H), 7,90 (d, 2H), 7,75 (m, 2H), 7,61 (d, 2H), 7,50 (s, 1 H), 6,89 (d, 1 H), 5,88 (s, 2H), 4,05 (t, 1 H), 3,15 (dd, 1 H), 3,03 (dd, 1 H), 1,32 (s, 9H).

### Beispiel 18 (nicht erfindungsgemäß)

### 3-(6-Amino-pyridin-3-yl)-2-{1-[5-(2-methyl-thiazol-4-yl)-isoxazol-3-yl-methyl]-1H-imidazol-4-yl}-propionsäure

Die Titelverbindung wurde analog zu Beispiel 2 als Trifluoressigsäuresalz aus 3-Brommethyl-5-(2-methyl-thiazol-4-yl)-isoxazol gewonnen.

LC/MS: Rₜ = 0,77 min, [M+H]⁺ = 411 (Methode A), ¹H-NMR (DMSO-d₆, 500 MHz): δ [ppm] = 13,7 (s, br, 1 H), 8,88 (s, br, 1 H), 8,20 (s, 1 H), 8,03 (s, br, 2H), 7,72 (m, 2H), 7,54 (s, 1 H), 6,92 (s, 1 H), 6,89 (d, 1 H), 5,55 (s, 2H), 4,10 (t, 1 H), 3,17 (dd, 1 H), 3,02 (dd, 1 H), 2,72 (s, 3H).

### Beispiel 19

### 3-(6-Amino-pyridin-3-yl)-2-{1-[5-(3,4-dichlor-phenyl)-isoxazol-3-ylmethyl]-1H-imidazol-4-yl}-propionsäure

Die Titelverbindung wurde analog zu Beispiel 2 als Trifluoressigsäuresalz aus 3-Brommethyl-5-(3,4-dichlor-phenyl)-isoxazol gewonnen.

LC/MS: Rₜ = 1,18 min, [M+H]⁺ = 458, Dichloropattern (Methode A). ¹H-NMR (DMSO-d₆, 500 MHz): δ [ppm] = 13,8 (s, br, 1 H), 8,83 (s, br, 1 H), 8,18 (s, 1 H), 8,01 (s, br, 2H), 7,86 (m, 2H), 7,72 (m, 2H), 7,54 (s, 1 H), 7,87 (d, 1 H), 5,58 (s, 2H), 4,10 (t, 1 H), 3,17 (dd, 1H), 3,02 (dd, 1 H).

### Beispiel 20

### 3-(6-Amino-pyridin-3-yl)-2-{1-[3-(4-methoxy-phenyl)-[1,2,4]oxadiazol-5-ylmethyl]-1H-imidazol-4-yl}-propionsäure

Die Titelverbindung wurde analog zu Beispiel 2 als Trifluoressigsäuresalz aus 5-Chlormethyl-3-(4-methoxy-phenyl)-[1,2,4]oxadiazol gewonnen.

LC/MS: Rₜ = 0,90 min, [M+H]⁺ = 421 (Methode A). ¹H-NMR (DMSO-d₆, 500 MHz): δ [ppm] = 13,5 (s, br, 1H), 8,56 (s, br, 1H), 8,01 (s, br, 2H), 7,91 (d, 2H), 7,76 (m, 2H), 7,49 (s, 1H), 7,11 (d, 2H), 6,88 (d, 1H), 5,82 (s, 2H), 4,07 (t, 1H), 3,82 (s, 3H), 3,15 (dd, 1H), 3,03 (dd, 1 H).

### Beispiel 21

### 3-(6-Amino-pyridin-3-yl)-2-[1-(5-phenyl-[1,3,4]thiadiazol-2-ylmethyl)-1 H-imidazol-4-yl]-propionsäure

Die Titelverbindung wurde analog zu Beispiel 2 als Trifluoressigsäuresalz aus 2-Brommethyl-5-phenyl-[1,3,4]thiadiazol gewonnen.

LC/MS: Rₜ = 0,81 min, [M+H]⁺ = 407 (Methode A). ¹H-NMR (DMSO-d₆, 500 MHz): δ [ppm] = 13,8 (s, br, 1 H), 8,78 (s, br, 1 H), 8,03 (s, br, 2H), 7,98 (d, 2H), 7,73 (m, 2H), 7,58 (m, 4H), 6,88 (d, 1 H), 5,91 (s, 2H), 4,09 (t, 1 H), 3,15 (dd, 1 H), 3,03 (dd, 1 H).

### Beispiel 22

### 3-(6-Amino-pyridin-3-yl)-2-{1-[5-(4-methoxy-phenyl)-[1,3,4]thiadiazol-2-ylmethyl]-1H-imidazol-4-yl}-propionsäure

Die Titelverbindung wurde analog zu Beispiel 2 als Trifluoressigsäuresalz aus 2-Brommethyl-5-(4-methoxy-phenyl)-[1,3,4]thiadiazol gewonnen.

LC/MS: Rₜ = 0,87 min, [M+H]⁺ = 437 (Methode A). ¹H-NMR (DMSO-d₆, 500 MHz): δ [ppm] = 13,8 (s, br, 1 H), 8,77 (s, br, 1 H), 8,02 (s, br, 2H), 7,91 (d, 2H), 7,72 (m, 2H), 7,52 (s, 1 H), 7,11 (d, 2H), 6,88 (d, 1 H), 5,88 (s, 2H), 4,08 (t, 1H), 3,85 (s, 3H), 3,15 (dd, 1H), 3,02 (dd, 1 H).

### Beispiel 23

### 3-(6-Amino-pyridin-3-yl)-2-{1-[5-(3,5-dimethyl-isoxazol-4-yl)-[1,2,4]oxadiazol-3-ylmethyl]-1H-imidazol-4-yl}-propionsäure

Die Titelverbindung wurde analog zu Beispiel 2 als Trifluoressigsäuresalz aus 3-Chlormethyl-5-(3,5-dimethyl-isoxazol-4-yl)-[1,2,4]oxadiazol gewonnen.

LC/MS: Rₜ = 0,72 min, [M+H]⁺ = 410 (Methode A). ¹H-NMR (DMSO-d₆, 500 MHz): δ [ppm] = 13,6 (s, br, 1 H), 8,60 (s, br, 1 H), 7,98 (s, br, 2H), 7,73 (m, 2H), 7,44 (s, 1 H), 6,86 (d, 1 H), 5,65 (s, 2H), 4,07 (t, 1 H), 3,13 (dd, 1 H), 3,03 (dd, 1 H), 2,72 (s, 3H), 2,48 (s, 3H).

### Beispiel 24

### 3-(6-Amino-pyridin-3-yl)-2-[1-(5-thiophen-2-yl-isoxazol-3-ylmethyl)-1H-imidazol-4-yl]-propionsäure

Die Titelverbindung wurde analog zu Beispiel 2 als Trifluoressigsäuresalz aus 3-Brommethyl-5-thiophen-2-isoxazol gewonnen.

LC/MS: Rₜ = 0,86 min, [M+H]⁺ = 396 (Methode B). ¹H-NMR (DMSO-d₆, 500 MHz): δ [ppm] = 13,9 (s, br, 1 H), 8,90 (s, br, 1 H), 8,02 (s, br, 2H), 8,86 (d, 1 H), 7,70 (m, 3H), 7,58 (s, 1 H), 7,25 (dd, 1 H), 6,89 (m, 2H), 5,53 (s, 2H), 4,10 (t, 1 H), 3,16 (dd, 1 H), 3,02 (dd, 1 H).

### Beispiel 25 (nicht erfindungsgemäß)

### 3-(6-Amino-pyridin-3-yl)-2-[1-(5-tert-butyl-1,2,4-oxadiazol-3-ylmethyl)-1 H-imidazol-4-yl]-propionsäure

Die Titelverbindung wurde analog zu Beispiel 2 als Trifluoressigsäuresalz aus 5-(tert-Butyl)-3-(Chlormethyl)-1,2,4-Oxadiazol gewonnen.

LC/MS: Rₜ = 0,77 min, [M+H]⁺ = 371 (Methode B). ¹H-NMR (DMSO-d₆, 500 MHz): δ [ppm] = 13,7 (s, br, 1H), 8,80 (s, br, 1 H), 8,06 (s, br, 2H), 7,72 (m, 2H), 7,51 (s, 1 H), 6,88 (d, 1 H), 5,59 (s, 2H), 4,42 (t, 1 H), 3,16 (dd, 1 H), 3,01 (dd, 1 H), 1,48 (s, 9H).

### Beispiel 26

### 3-(6-Amino-pyridin-3-yl)-2-(1-{3-[4-(4-chlor-benzyloxy)-phenyl]-[1,2,4]oxadiazol-5-ylmethyl}-1H-imidazol-4-yl)-propionsäure

Die Titelverbindung wurde analog zu Beispiel 2 als Trifluoressigsäuresalz aus 3-[4-(4-Chlor-benzyloxy)-phenyl]-5-chlormethyl-[1,2,4]oxadiazol gewonnen.

LC/MS: Rₜ = 1,36 min, [M+H]⁺ = 371, Chloropattern, (Methode A).

Beispiel 27 (nicht erfindungsgemäß)

### 3-(6-Amino-pyridin-3-yl)-2-[1-(4-brom-thiophen-2-ylmethyl)-1 H-imidazol-4-yl]-propionsäure

Die Titelverbindung wurde analog zu Beispiel 2 als Trifluoressigsäuresalz aus Methansulfonsäure-4-brom-thiophen-2-ylmethylester gewonnen.

LC/MS: Rₜ = 0,80 min, [M+H]⁺ = 407, Bromopattern (Methode A).

### Beispiel 28 (nicht erfindungsgemäß)

### 3-(6-Amino-pyridin-3-yl)-2-[1-(5-methyl-isoxazol-3-ylmethyl)-1H-imidazol-4-yl]-propionsäure

Die Titelverbindung wurde analog zu Beispiel 2 als Trifluoressigsäuresalz aus 3-Brommethyl-5-methyl-isoxazol gewonnen.

LC/MS: Rₜ = 0,52 min, [M+H]⁺ = 328 (Methode A).

### Beispiel 29

### 3-(6-Amino-pyridin-3-yl)-2-[1-(4-phenyl-5-trifluormethyl-thiophen-2-ylmethyl)-1H-imidazol-4-yl]-propionsäure

Die Titelverbindung wurde analog zu Beispiel 2 als Trifluoressigsäuresalz aus Methansulfonsäure-4-phenyl-5-trifluormethyl-thiophen-2-ylmethylester gewonnen. LC/MS: Rₜ = 1,36 min, [M+H]⁺ = 473 (Methode A).

### Beispiel 30

### 3-(6-Amino-pyridin-3-yl)-2-{1-(5-(4-brom-phenyl)-isoxazol-3-ylmethyl]-1H-imidazol-4-yl}-propionsäure

Die Titelverbindung wurde analog zu Beispiel 2 als Trifluoressigsäuresalz aus Methansulfonsäure-5-(4-brom-phenyl)-isoxazol-3-ylmethylester gewonnen.

LC/MS: Rₜ = 1,11 min, [M+H]⁺ = 468, Bromopattern (Methode A).

### Beispiel 31

### 3-(6-Amino-pyridin-3-yl)-2-[1-(5-p-tolyl-isoxazol-3-ylmethyl)-1H-imidazol-4-yl]-propionsäure

Die Titelverbindung wurde analog zu Beispiel 2 als Trifluoressigsäuresalz aus Methansulfonsäure-5-(4-methyl-phenyl)-isoxazol-3-ylmethylester gewonnen.

LC/MS: Rₜ = 1,07 min, [M+H]⁺ = 404 (Methode A).

### Beispiel 32

### 3-(6-Amino-pyridin-3-yl)-2-(1-{2-[5-(5-chlor-thiophen-2-yl)-isoxazol-3-yl]-ethyl}-1H-imidazol-4-yl)-propionsäure

Die Titelverbindung wurde analog zu Beispiel 2 als Trifluoressigsäuresalz aus Toluol-4-sulfonsäure-2-[5-(5-chlor-thiophen-2-yl)-isoxazol-3-yl]-ethylester gewonnen.

LC/MS: Rₜ = 1,05 min, [M+H]⁺ = 444, Chloropattern (Methode A).

### Beispiel 33

### 3-(6-Amino-pyridin-3-yl)-2-{1-[5-(4-isobutyl-phenyl)-isoxazol-3-ylmethyl]-1H-imidazol-4-yl}-propionsäure

Die Titelverbindung wurde analog zu Beispiel 2 als Trifluoressigsäuresalz aus Methansulfonsäure-5-(4-isobutyl-phenyl)-isoxazol-3-ylmethylester gewonnen.

LC/MS: Rₜ = 1,29 min, [M+H]⁺ = 446 (Methode A).

### Beispiel 34

### 3-(6-Amino-pyridin-3-yl)-2-[1-(5-cyclopentyl-[1,3,4]thiadiazol-2-ylmethyl)-1 H-imidazol-4-yl]-propionsäure

Die Titelverbindung wurde analog zu Beispiel 2 als Trifluoressigsäuresalz aus Methansulfonsäure-5-cyclopentyl-[1,3,4]thiadiazol-2-ylmethylester gewonnen.

LC/MS: Rₜ = 0,82 min, [M+H]⁺ = 399 (Methode A).

### Beispiel 35

### 3-(6-Amino-pyridin-3-yl)-2-[1-(5-cyclobutyl-[1,3,4]thiadiazol-2-ylmethyl)-1H-imidazol-4-yl]-propionsäure

Die Titelverbindung wurde analog zu Beispiel 2 als Trifluoressigsäuresalz aus Methansulfonsäure-5-cyclobutyl-[1,3,4]thiadiazol-2-ylmethylester gewonnen.

LC/MS: Rₜ = 0,71 min, [M+H]⁺ = 385 (Methode A).

### Beispiel 36

### 3-(6-Amino-pyridin-3-yl)-2-[1-(5-cyclopropyl-isoxazol-3-ylmethyl)-1 H-imidazol-4-yl]-propionsäure

Die Titelverbindung wurde analog zu Beispiel 2 als Trifluoressigsäuresalz aus Methanesulfonsäure-5-cyclopropyl-isoxazol-3-ylmethyl-ester gewonnen.

LC/MS: Rₜ = 0,69 min, [M+H]⁺ = 354 (Methode A).

### Beispiel 37

### 3-(6-Amino-pyridin-3-yl)-2-[1-(5-cyclohexyl-isoxazol-3-ylmethyl)-1 H-imidazol-4-yl]-propionsäure

Die Titelverbindung wurde analog zu Beispiel 2 als Trifluoressigsäuresalz aus Methanesulfonsäure-5-cyclohexyl-isoxazol-3-ylmethyl-ester gewonnen.

LC/MS: Rₜ = 1,07 min, [M+H]⁺ = 396 (Methode A).

### Beispiel 38

### 3-(6-Amino-pyridin-3-yl)-2-{1-[5-cyclohexyl-[1,3,4]thiadiazol-2-ylmethyl]-1H-imidazol-4-yl}-propionsäure

Die Titelverbindung wurde analog zu Beispiel 2 als Trifluoressigsäuresalz aus Methansulfonsäure-5-cyclohexyl-[1,3,4]thiadiazol-2-ylmethyl-ester gewonnen. LC/MS: Rₜ = 0,90 min, [M+H]⁺ = 413 (Methode A).

### Beispiel 39 (nicht erfindungsgemäß)

### 3-(6-Amino-pyridin-3-yl)-2-{1-[5-(3-methyl-butyl)-[1,3,4]thiadiazol-2-ylmethyl]-1H-imidazol-4-yl}-propionsäure

Die Titelverbindung wurde analog zu Beispiel 2 als Trifluoressigsäuresalz aus Methansulfonsäure-5-(3-methyl-butyl)-[1,3,4]thiadiazol-2-ylmethyl-ester gewonnen. LC/MS: Rₜ = 0,92 min, [M+H]⁺ = 401 (Methode A).

### Beispiel 40

### 3-(6-Amino-pyridin-3-yl)-2-[1-(5-cyclobutyl-isoxazol-3-ylmethyl)-1 H-imidazol-4-yl]-propionsäure

Die Titelverbindung wurde analog zu Beispiel 2 als Trifluoressigsäuresalz aus Methansulfonsäure-5-cyclobutyl-isoxazol-3-ylmethyl-ester gewonnen.

LC/MS: Rₜ = 0,89 min, [M+H]⁺ = 368 (Methode A).

### Beispiel 41

### 3-(6-Amino-pyridin-3-yl)-2-{1-[5-(4-fluor-phenyl)-isoxazol-3-ylmethyl]-1H-imidazol-4-yl}-propionsäure

Die Titelverbindung wurde analog zu Beispiel 2 als Trifluoressigsäuresalz aus Methansulfonsäure-5-(4-fluor-phenyl)-isoxazol-3-ylmethyl-ester gewonnen.

LC/MS: Rₜ = 1,04 min, [M+H]⁺ = 408 (Methode A).

### Beispiel 42

### 3-(6-Amino-pyridin-3-yl)-2-{1-[5-(4-benzyl-phenyl)-isoxazol-3-ylmethyl]-1H-imidazol-4-yl}-propionsäure

Die Titelverbindung wurde analog zu Beispiel 2 als Trifluoressigsäuresalz aus Methansulfonsäure-5-(4-benzyl-phenyl)-isoxazol-3-ylmethylester gewonnen.

LC/MS: Rₜ = 1,28 min, [M+H]⁺ = 480 (Methode A).

### Beispiel 43

### 3-(6-Amino-pyridin-3-yl)-2-{1-[5-(4-tert-butyl-phenyl)-isoxazol-3-ylmethyl]-1H-imidazol-4-yl}-propionsäure

Die Titelverbindung wurde analog zu Beispiel 2 als Trifluoressigsäuresalz aus Methansulfonsäure-5-(4-tert-butyl-phenyl)-isoxazol-3-ylmethyl-ester gewonnen. LC/MS: Rₜ = 1,28 min, [M+H]⁺ = 446 (Methode A).

### Beispiel 44

### 3-(6-Amino-pyridin-3-yl)-2-{1-[5-(4-tert-butyl-2,6-dimethyl-phenyl)-isoxazol-3-ylmethyl]-1H-imidazol-4-yl}-propionsäure

Die Titelverbindung wurde analog zu Beispiel 2 als Trifluoressigsäuresalz aus Methansulfonsäure-5-(4-tert-butyl-2,6-dimethyl-phenyl)-isoxazol-3-ylmethyl ester gewonnen. LC/MS: Rₜ = 1,34 min, [M+H]⁺ = 474 (Methode A).

### Beispiel 45

### 3-(6-Amino-pyridin-3-yl)-2-{1-[5-(2-chloro-phenyl)-isoxazol-3-ylmethyl]-1H-imidazol-4-yl}-propionsäure

Die Titelverbindung wurde analog zu Beispiel 2 als Trifluoressigsäuresalz aus Methansulfonsäure-5-(2-chloro-phenyl)-isoxazol-3-ylmethyl ester gewonnen.

LC/MS: Rₜ = 1,05 min, [M+H]⁺ = 424, Chloropattern (Methode A).

### Beispiel 46

### 3-(6-Amino-pyridin-3-yl)-2-{1-[5-(4-sec-butyl-phenyl)-isoxazol-3-ylmethyl]-1H-imidazol-4-yl}-propionsäure

Die Titelverbindung wurde analog zu Beispiel 2 als Trifluoressigsäuresalz aus Methansulfonsäure-5-(4-sec-butyl-phenyl)-isoxazol-3-ylmethyl-ester gewonnen. LC/MS: Rₜ = 1,29 min, [M+H]⁺ = 446 (Methode A).

### Beispiel 47

### 3-(6-Amino-pyridin-3-yl)-2-[1-(5-indan-5-yl-isoxazol-3-ylmethyl)-1 H-imidazol-4-yl]-propionsäure

Die Titelverbindung wurde analog zu Beispiel 2 als Trifluoressigsäuresalz aus Methanesulfonsäure-5-indan-5-yl-isoxazol-3-ylmethyl-ester gewonnen.

LC/MS: Rₜ = 1,15 min, [M+H]⁺ = 430 (Methode A).

### Beispiel 48

### 3-(6-Amino-pyridin-3-yl)-2-{1-[5-(4-cyclopentyl-phenyl)-isoxazol-3-ylmethyl]-1H-imidazol-4-yl}-propionsäure

Die Titelverbindung wurde analog zu Beispiel 2 als Trifluoressigsäuresalz aus Methansulfonsäure-5-(4-cyclopentyl-phenyl)-isoxazol-3-ylmethylester gewonnen. LC/MS: Rₜ = 1,30 min, [M+H]⁺ = 458 (Methode A).

### Beispiel 49

### 3-(6-Amino-pyridin-3-yl)-2-{1-[5-(4-isopropyl-phenyl)-isoxazol-3-ylmethyl]-1H-imidazol-4-yl}-propionsäure

Die Titelverbindung wurde analog zu Beispiel 2 als Trifluoressigsäuresalz aus Methansulfonsäure-5-(4-isopropyl-phenyl)-isoxazol-3-ylmethyl-ester gewonnen. LC/MS: Rₜ = 1,21 min, [M+H]⁺ = 432 (Methode A).

### Beispiel 50

### 3-(6-Amino-pyridin-3-yl)-2-{1-[5-(4-butyl-phenyl)-isoxazol-3-ylmethyl]-1H-imidazol-4-yl}-propionsäure

Die Titelverbindung wurde analog zu Beispiel 2 als Trifluoressigsäuresalz aus Methansulfonsäure-5-(4-butyl-phenyl)-isoxazol-3-ylmethyl-ester gewonnen.

LC/MS: Rₜ = 1,30 min, [M+H]⁺ = 446 (Methode A).

### Beispiel 51

### 3-(6-Amino-pyridin-3-yl)-2-{1-[5-(4-cyclohexyl-phenyl)-isoxazol-3-ylmethyl]-1H-imidazol-4-yl}-propionsäure

Die Titelverbindung wurde analog zu Beispiel 2 als Trifluoressigsäuresalz aus Methansulfonsäure-5-(4-cyclohexyl-phenyl)-isoxazol-3-ylmethyl-ester gewonnen.

LC/MS: Rₜ = 1,38 min, [M+H]⁺ = 472 (Methode A).

### Beispiel 52

### 3-(6-Amino-pyridin-3-yl)-2-{1-[5-(5,6,7,8-tetrahydro-naphthalen-2-yl)-isoxazol-3-ylmethyl]-1H-imidazol-4-yl}-propionsäure

Die Titelverbindung wurde analog zu Beispiel 2 als Trifluoressigsäuresalz aus Methansulfonsäure-5-(5,6,7,8-tetrahydro-naphthalen-2-yl)-isoxazol-3-ylmethyl-ester gewonnen. LC/MS: Rₜ = 1,23 min, [M+H]⁺ = 444 (Methode A).

### Beispiel 53

### 3-(6-Amino-pyridin-3-yl)-2-{1-[5-(4-propyl-phenyl)-isoxazol-3-ylmethyl]-1H-imidazol-4-yl}-propionsäure

Die Titelverbindung wurde analog zu Beispiel 2 als Trifluoressigsäuresalz aus Methansulfonsäure-5-(4-propyl-phenyl)-isoxazol-3-ylmethyl-ester gewonnen.

LC/MS: Rₜ = 1,32 min, [M+H]⁺ = 432 (Methode A).

### Beispiel 54

### 3-(6-Amino-pyridin-3-yl)-2-{1-[5-(4-phenethyl-phenyl)-isoxazol-3-ylmethyl]-1H-imidazol-4-yl}-propionsäure

Die Titelverbindung wurde analog zu Beispiel 2 als Trifluoressigsäuresalz aus Methansulfonsäure-5-(4-phenethyl-phenyl)-isoxazol-3-ylmethyl-ester gewonnen.

LC/MS: Rₜ = 1,37 min, [M+H]⁺ = 494 (Methode B).

### Beispiel 55

### 3-(6-Amino-pyridin-3-yl)-2-{1-[5-(2,3-dihydro-benzo[1,4]dioxin-6-yl)-isoxazol-3-ylmethyl]-1H-imidazol-4-yl}-propionsäure

Die Titelverbindung wurde analog zu Beispiel 2 als Trifluoressigsäuresalz aus Methansulfonsäure-5-(2,3-dihydro-benzo[1,4]dioxin-6-yl)-isoxazol-3-ylmethyl-ester gewonnen. LC/MS: Rₜ = 0,87 min, [M+H]⁺ = 448 (Methode A).

### Beispiel 56

### (S)-3-(6-Amino-pyridin-3-yl)-2-{1-[5-(5-chlorthiophen-2-yl)-isoxazol-3-ylmethyl]-1H-imidazol-4-yl}-propionsäureethylester

Eine Lösung von 5,0 g (19,21 mmol) 3-(6-Aminopyridin-3-yl)-2-(1 H-imidazol-4-yl)-propionsäure in 200 mL absoluten DMF wurde unter Argon mit 0,485g (60%ig in Mineralöl, 19,21 mmol) Natriumhydrid versetzt. Man ließ 30 min bei RT rühren, gibt dann 5,643g (19,21 mmol) Methansulfonsäure-5-(5-chloro-thiophen-2-yl)-isoxazol-3-ylmethylester zu und ließ erneut 3 h bei RT rühren. Das Reaktionsgemisch wurde unter verminderten Druck eingeengt und der erhaltene Rückstand wurde an Kieselgel mit CH₂Cl₂/MeOH/Wasser/Essigsäure 90:10:1:1 chromatographiert. Es wurden zwei produkthaltige Fraktionen erhalten, die über präparative HPLC gereinigt wurden. Die Wertfraktionen wurden vereinigt und gefriergetrocknet. Der erhaltene Rückstand wurde in Ethylacetat gelöst, mit gesättigter NaHCO₃-Lösung gewaschen, über Na₂SO₄ getrocknet, filtriert und eingeengt. Das so erhaltene Material (3,7g) wurde durch chirale HPLC in seine Enantiomeren getrennt (Säule Chiralpak AD-H/55, 250x4,6mm, Fluß 1mL/min, Eluens Heptan:i-Propanol:Methanol 3:1:1+0,1% TFA). 0,1g (218 µmol) des erhaltenen Produkts wurden in 5 mL Wasser/Essigsäure (1:1) gelöst und gefriergetrocknet (0,108g, 95%).

LC/MS: Rₜ = 1,14 min, [M+H]⁺ = 458, Chloropattern (Methode A).

Chirale HPLC: Rₜ = 5,312 min.

### Beispiel 57

### (R)-3-(6-Amino-pyridin-3-yl)-2-{1-[5-(5-chlorthiophen-2-yl)-isoxazol-3-ylmethyl]-1H-imidazol-4-yl}-propionsäureethylester

Die Titelverbindung wird als zweites Enantiomer bei der Herstellung von Beispiel 56 erhalten.

LC/MS: Rₜ = 1,14 min, [M+H]⁺ = 458, Chloropattern (Methode A).

Chirale HPLC: Rₜ = 8,443 min.

### Beispiel 58

### (S)-3-(6-Amino-pyridin-3-yl)-2-{1-[5-(5-chlor-thiophen-2-yl)-isoxazol-3-ylmethyl]-1H-imidazol-4-yl}-propionsäure

Eine Lösung von 0,11g (0,24 mmol) (S)-3-(6-Amino-pyridin-3-yl)-2-{1-[5-(5-chlorthio-phen-2-yl)-isoxazol-3-ylmethyl]-1H-imidazol-4-yl}-propionsäureethylester in 2 mL halbkonzentrierter Salzsäure wurde 24 h bei RT gerührt. Das Reaktionsgemisch wurde gefriergetrocknet und über präparative HPLC gereinigt. Die erhaltenen Wertfraktionen wurden vereinigt und mit 2 Äquivalenten (eq) 1N HCl (1 x) und mit 1 eq 1 N HCl und Wasser (1x) gefriergetrocknet. Man erhielt 0,077g (69%) der Titelverbindung als Hydrochlorid.

LC/MS: Rₜ = 1,07 min, [M+H]⁺ = 430, Chloropattern (Methode A).

### Beispiel 59

### (R)-3-(6-Amino-pyridin-3-yl)-2-{1-[5-(5-chlor-thiophen-2-yl)-isoxazol-3-ylmethyl]-1H-imidazol-4-yl}-propionsäure

Die Titelverbindung wurde analog zu Beispiel 58 als Hydrochlorid erhalten.

LC/MS: Rₜ = 1,02 min, [M+H]⁺ = 430, Chloropattern (Methode A).

### Beispiel 60 (nicht erfindungsgemäß)

### 6-Amino-2-[1-(5-cyclopropyl-[1,3,4]thiadiazol-2-ylmethyl)-1 H-imidazol-4-yl]-hexansäure

### a) 2-[1-(Toluol-4-sulfonyl)-1H-imidazol-4-yl]-malonsäurediethylester

Eine Lösung von 1,000g (3,243 mmol) [1-(Toluol-4-sulfonyl)-1 H-imidazol-4-yl]-essigsäureethylester wurde in 20 mL THF gelöst und auf 0 °C gekühlt. Innerhalb von 10 min wurden 3,567 mL Lithiumbis(trimethylsilyl)amid (3,567 mmol, 1 M in THF) zugetropft. Man ließ 30 min bei 0 °C nachrühren, gab dann 350 µL (3,567 mmol) Cyanameisensäureethylester zu und ließ 2 h bei RT nachrühren. Das Reaktionsgemisch wurde mit gesättigter Ammoniumchlorid-Lösung gequencht und mit Ethylacetat extrahiert. Die organische Phase wurde mit Wasser und gesättigter NaCl-Lösung gewaschen, über Na₂SO₄ getrocknet, filtriert und eingeengt. Chromatographie an Kieselgel (Heptan/Ethylacetat 1:1) liefert die Titelverbindung (0,390g, 32%). LC/MS: Rₜ = 1,74 min, [M+H]⁺ = 381, (Methode A).

### b) 2-(4-tert-Butoxycarbonylamino-butyl)-2-[1-(toluol-4-sulfonyl)-1H-imidazol-4-yl]-malonsäurediethylester

Eine Lösung von 4,0 g (10,51 mmol) 2-[1-(Toluol-4-sulfonyl)-1H-imidazol-4-yl]-malonsäurediethylester in 67mL DMF wurde mit 13,7 g (42,0 mmol) Cäsiumcarbonat und 3,24g (11,56 mmol) 4-(t-Butyloxycarbonyl-amino)-butylbromid versetzt und für 2 h bei 60 °C gerührt. Das Reaktionsgemisch wurde unter vermindertem Druck eingeengt, der Rückstand in Ethylacetat aufgenommen und mit Wasser gewaschen. Die organische Phase wurde über Na₂SO₄ getrocknet, filtriert und eingeengt. Chromatographie an Kieselgel (Heptan/Ethylacetat 1:1) liefert die Titelverbindung (4,21g, 73%). LC/MS: Rₜ = 2,08 min, [M+H]⁺ = 552, (Methode A).

### c) 2-(4-tert-Butoxycarbonylamino-butyl)-2-(1H-imidazol-4-yl)-malonsäurediethylester

Man löste 4,2 g (7,61 mmol) 2-(4-tert-Butoxycarbonylamino-butyl)-2-[1-(toluol-4-sulfonyl)-1H-imidazol-4-yl]-malonsäurediethylester in 190 mL Methanol und rührte mit 4,66g (30,45 mmol) 1-Hydroxybenzotriazol-Hydrat für 1 h bei RT. Das Reaktionsgemisch wurde unter vermindertem Druck eingeengt und der Rückstand an Kieselgel chromatogaphiert (CH₂Cl₂:MeOH 9:1). Die produkthaltigen Fraktionen wurden eingeengt, das erhaltene Öl wurde in Ethylacetat gelöst und mit gesättigter NaHCO₃-Lösung gewaschen. Die organische Phase wurde über Na₂SO₄ getrocknet, filtriert und eingeengt. Man erhielt 2,14g (71%) der Titelverbindung.

LC/MS: Rₜ = 1,26 min, [M+H]⁺ = 398, (Methode A).

### d) 2-(4-tert-Butoxycarbonylamino-butyl)-2-[1-(5-cyclopropyl-[1,3,4]thiadiazol-2-ylmethyl)-1 H-imidazol-4-yl]-malonsäurediethylester

Eine Lösung von 0,60g (1,51 mmol) 2-(4-tert-Butoxycarbonylamino-butyl)-2-(1H-imidazol-4-yl)-malonsäurediethylester in 12 mL DMF wurde mit 1,97g (6,04 mmol) Cäsiumcarbonat und 0,29g (1,66 mmol) 2-Chlormethyl-5-cyclopropyl-1,3,4-thiadiazol für 2 h auf 75 °C erwärmt. Das Reaktionsgemisch wurde unter vermindertem Druck eingeengt, der Rückstand wurde in Ethylacetat aufgenommen und mit Wasser gewaschen. Die organische Phase wurde über Na₂SO₄ getrocknet, filtriert und eingeengt. Chromatographische Reinigung des Rückstandes an Kieselgel (CH₂Cl₂:MeOH:Wasser:Essigsäure 95:5:0,5:0,5) lieferte 0,145g (18%) der Titelverbindung. LC/MS: Rₜ = 1,40 min, [M+H]⁺ = 536, (Methode A).

### e) 6-Amino-2-[1-(5-cyclopropyl-[1,3,4]thiadiazol-2-ylmethyl)-1H-imidazol-4-yl]-hexansäure.

Man suspendierte 0,140g (261 µmol) 2-(4-tert-Butoxycarbonylamino-butyl)-2-[1-(5-cyclopropyl-[1,3,4]thiadiazol-2-ylmethyl)-1H-imidazol-4-yl]-malonsäurediethylester in 10 mL halbkonzentrierter Salzsäure und erhitzte für 8 h unter Rühren auf 95 °C. Das Reaktionsgemisch wurde unter vermindertem Druck eingeengt, durch präparative HPLC gereinigt und die produkthaltigen Fraktionen wurden gefriergetrocket, anschließend einmal nach Aufnehmen in 2,5 eq 1 N Salzsäure und einmal nach Aufnehmen in Wasser gefriergetrocknet. Das Präparat wurde anschließend nochmals über präparative HPLC gereinigt und die produkthaltigen Fraktionen wurden gefriergetrocknet. Man erhielt 35 mg (32%) der Titelverbindung als Trifluoressigsäuresalz.

LC/MS: Rₜ = 0,34 min, [M+H]⁺ = 336, (Methode A).

### Beispiel 61

### 3-(6-Amino-5-methyl-pyridin-3-yl)-2-[1-(5-cyclopropyl-[1,3,4]thiadiazol-2-ylmethyl)-1 H-imidazol-4-yl]-propionsäure

Die Titelverbindung wurd analog zu Beispiel 60 hergestellt.

LC/MS: Rₜ = 0,50 min, [M+H]⁺ = 385 (Methode B).

Beispiel 62 (nicht erfindungsgemäß)

### 3-(4-Amino-cyclohexyl)-2-[1-(5-cyclopropyl-[1,3,4]thiadiazol-2-ylmethyl)-1 H-imidazol-4-yl]-propionsäure

Die Titelverbindung wurde analog zu Beispiel 60 hergestellt.

LC/MS: Rₜ = 0,64 min, [M+H]⁺ = 376 (Methode A).

### Beispiel 63 (nicht erfindungsgemäß)

### 3-(4-Amino-cyclohexyl)-2-[1-(5-cyclopropyl-[1,3,4]thiadiazol-2-ylmethyl)-1 H-imidazol-4-yl]-propionsäure

Die Titelverbindung wurde analog zu Beispiel 60 hergestellt.

LC/MS: Rₜ = 0,71 min, [M+H]⁺ = 376 (Methode A).

### Pharmakologische Beispiele

Die hergestellten Substanzen wurden mit dem Actichrome Plasma TAFI Activity Kit der Firma American Diagnostica (Pr.Nr. 874) auf Inhibition von TAFIa geprüft. Dabei wurden zu 1 µl 5 mM DMSO-Lösung der Substanz 29 µL Testpuffer (20 mM Hepes, 150 mM NaCl, pH 7,4) und 10 µL TAFIa (American Diagnostica Pr.Nr. 874TAFIA; 2.5 /ml) gegeben und 15 Minuten bei Raumtemperatur in einer 96 half-well Mikrotiterplatte inkubiert. Die Enzymreaktion wurde durch Zugabe von 10 µL TAFIa "Developer" (1:2 mit Wasser vorverdünnt) gestartet. Der Zeitverlauf der Reaktion wurde bei 420 nm in einem Mikrotiterplattenreader (SpectraMax plus 384; Fa. Molecular Devices) über 15 Minuten verfolgt.

Die IC₅₀ wurde aus den gemittelten Werten (Doppelbestimmung) einer Verdünnungsreihe der Substanz mit Hilfe der Software Grafit 4 (Erithacus Software, UK) berechnet. Tabelle 1 zeigt die Ergebnisse.

**Tabelle 1:**

| Verbindung aus Beispiel | TAFIa-Enzym-Assay | Verbindung aus Beispiel | TAFIa-Enzym-Assay |
|---|---|---|---|
| | IC₅₀ [mikro M] | | IC₅₀ [mikro M] |
| 1 | 0,041 | 15 | 0,223 |
| 3 | 0,326 | 17 | 0,745 |
| 5 | 0,104 | 18 | 0,326 |
| 8 | 0,041 | 22 | 0,078 |
| 9 | 0,169 | 46 | 0,003 |
| 10 | 0,258 | 58 | 0,004 |
| 12 | 0,030 | 61 | 0,026 |

## Patentansprüche

1. Verbindung der Formel I 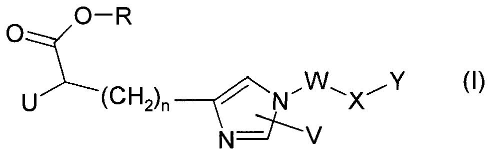 und/oder alle stereoisomeren Formen der Verbindung der Formel I und/oder Gemische dieser Formen in jedem Verhältnis, und/oder ein physiologisch verträgliches Salz der Verbindung der Formel I, wobei
U für -(C₁-C₄)-Alkylen-Het-Z steht und wobei
U und Z zusammen den Rest 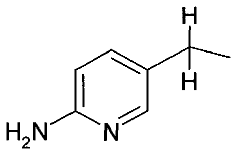 darstellen und der Pyridylteil im Rest unsubstituiert oder durch Methyl oder Ethyl substituiert ist,
n für Null steht,
R für Wasserstoffatom oder -(C₁-C₄)-Alkyl steht,
V für
1) Wasserstoffatom,
2) -(C₁-C₃)-Alkyl oder
3) Fluor, Chlor oder Brom steht,
W für -(C₁-C₃)-Alkylen steht,
X für einen aromatischen fünf- bis dreizehngliedrigen Heterocyclus steht, wobei Heterocyclus ausgewählt ist aus der Gruppe Isoxazolyl, 1,2,3-Oxadiazolyl, 1,2,4-Oxadiazolyl, 1,2,5-Oxadiazolyl, 1,3,4-Oxadiazolyl, Oxazolyl, 1,2,3-Thiadiazolyl, 1,2,4-Thiadiazolyl, 1,2,5-Thiadiazolyl, 1,3,4-Thiadiazolyl, Thienopyridin oder Thienyl, und Heterocyclus unsubstituiert oder ein-, zwei- oder dreifach unabhängig voneinander durch R1 substituiert ist,
Y für
1) -(C₃-C₆)-Cycloalkyl, wobei Cycloalkyl unsubstituiert oder ein-, zwei- oder dreifach unabhängig voneinander durch R1 substituiert ist,
2) -(C₆-C₁₄)-Aryl, wobei Aryl ausgewählt ist aus der Gruppe Indanyl, Naphthyl, Phenyl oder Tetrahydronaphthalenyl, wobei Aryl unsubstituiert oder ein-, zwei- oder dreifach unabhängig voneinander durch R1 substituiert ist, oder
3) Heterocyclus steht, wobei Heterocyclus ausgewählt ist aus der Gruppe 2,3-Dihydro-benzo[1,4]dioxin, Isoxazolyl, 1,2,3-Oxadiazolyl, 1,2,4-Oxadiazolyl, 1,2,5-Oxadiazolyl, 1,3,4-Oxadiazolyl, Oxazolyl, 1,2,3-Thiadiazolyl, 1,2,4-Thiadiazolyl, 1,2,5-Thiadiazolyl, 1,3,4-Thiadiazolyl, Thienopyridin oder Thienyl, und Het unsubstituiert oder ein-, zwei- oder dreifach unabhängig voneinander durch R1 substituiert ist, und
R1 für Fluor, Chlor, Brom, -(C₁-C₄)-Alkyl, -(C₀-C₄)-Alkylen-Phenyl, -O-CH₃, -O-(C₁-C₄)-Alkylen-Phenyl, wobei Phenyl unsubstituiert oder ein-, oder zweifach durch Fluor, Chlor, Brom oder -O-(C₁-C₄)-Alkyl substituiert ist, -(C₃-C₆)-Cycloalkyl oder -CF₃ steht.

2. Verbindung der Formel I gemäß Anspruch 1, **dadurch gekennzeichnet, dass** es die Verbindung
3-(6-Amino-pyridin-3-yl)-2-{1-[5-(5-chlor-thiophen-2-yl)-isoxazol-3-ylmethyl]-1H-imidazol-4-yl}-propionsäure,
3-(6-Amino-pyridin-3-yl)-2-[1-(5-phenyl-isoxazol-3-ylmethyl)-1 H-imidazol-4-yl]-propionsäure,
3-(6-Amino-pyridin-3-yl)-2-{1-[5-(4-methoxy-phenyl)-isoxazol-3-ylmethyl]-1H-imidazol-4-yl}-propionsäure,
3-(6-Amino-pyridin-3-yl)-2-{1-[5-(5-chlor-thiophen-2-yl)-[1,3,4]thiadiazol-2-ylmethyl]-1H-imidazol-4-yl}-propionsäure,
3-(6-Amino-pyridin-3-yl)-2-{1-[5-(4-chlor-phenyl)-[1,3,4]thiadiazol-2-ylmethyl]-1H-imidazol-4-yl}-propionsäure,
3-(6-Amino-pyridin-3-yl)-2-{1-[5-(4-trifluormethyl-phenyl)-[1,2,4]oxadiazol-3-ylmethyl]-1H-imidazol-4-yl}-propionsäure,
3-(6-Amino-pyridin-3-yl)-2-{1-[5-(3-trifluormethyl-phenyl)-[1,2,4]oxadiazol-3-ylmethyl]-1H-imidazol-4-yl}-propionsäure,
3-(6-Amino-pyridin-3-yl)-2-[1-(5-cyclopropyl-[1,3,4]thiadiazol-2-ylmethyl)-1H-imidazol- 4-yl]-propionsäure,
3-(6-Amino-pyridin-3-yl)-2-[1-(5-phenyl-[1,2,4]oxadiazol-3-ylmethyl)-1H-imidazol-4-yl]-propionsäure,
3-(6-Amino-pyridin-3-yl)-2-{1-[3-(3,4-dimethoxy-phenyl)-[1,2,4]oxadiazol-5-ylmethyl]-1H-imidazol-4-yl}-propionsäure,
3-(6-Amino-pyridin-3-yl)-2-{1-[3-(4-methoxy-phenyl)-[1,2,4]oxadiazol-5-ylmethyl]-1H-imidazol-4-yl}-propionsäure,
3-(6-Amino-pyridin-3-yl)-2-{1-[3-(4'-isopropyl-phenyl)-[1,2,4]oxadiazol-5-ylmethyl]-1H-imidazol-4-yl}-propionsäure,
3-(6-Amino-pyridin-3-yl)-2-{1-[3-(4'-tert-butyl-phenyl)-[1,2,4]oxadiazol-5-ylmethyl]-1H-imidazol-4-yl}-propionsäure,
3-(6-Amino-pyridin-3-yl)-2-{1-[5-(3,4-dichlor-phenyl)-isoxazol-3-ylmethyl]-1H-imidazol-4-yl}-propionsäure,
3-(6-Amino-pyridin-3-yl)-2-{1-[3-(4-methoxy-phenyl)-[1,2,4]oxadiazol-5-ylmethyl]-1H-imidazol-4-yl}-propionsäure,
3-(6-Amino-pyridin-3-yl)-2-[1-(5-phenyl-[1,3,4]thiadiazol-2-ylmethyl)-1H-imidazol-4-yl]-propionsäure,
3-(6-Amino-pyridin-3-yl)-2-{1-[5-(4-methoxy-phenyl)-[1,3,4]thiadiazol-2-ylmethyl]-1H-imidazol-4-yl}-propionsäure,
3-(6-Amino-pyridin-3-yl)-2-{1-[5-(3,5-dimethyl-isoxazol-4-yl)-[1,2,4]oxadiazol-3-ylmethyl]-1H-imidazol-4-yl}-propionsäure,
3-(6-Amino-pyridin-3-yl)-2-[1-(5-thiophen-2-yl-isoxazol-3-ylmethyl)-1 H-imidazol-4-yl]-propionsäure,
3-(6-Amino-pyridin-3-yl)-2-(1-{3-[4-(4-chlor-benzyloxy)-phenyl]-[1,2,4]oxadiazol-5-ylmethyl}-1H-imidazol-4-yl)-propionsäure,
3-(6-Amino-pyridin-3-yl)-2-[1-(4-phenyl-5-trifluormethyl-thiophen-2-ylmethyl)-1H-imidazol-4-yl]-propionsäure,
3-(6-Amino-pyridin-3-yl)-2-{1-[5-(4-brom-phenyl)-isoxazol-3-ylmethyl]-1H-imidazol-4-yl}-propionsäure,
3-(6-Amino-pyridin-3-yl)-2-[1-(5-p-tolyl-isoxazol-3-ylmethyl)-1 H-imidazol-4-yl]-propionsäure,
3-(6-Amino-pyridin-3-yl)-2-(1-{2-[5-(5-chlor-thiophen-2-yl)-isoxazol-3-yl]-ethyl}-1 H-imidazol-4-yl)-propionsäure,
3-(6-Amino-pyridin-3-yl)-2-{1-[5-(4-isobutyl-phenyl)-isoxazol-3-ylmethyl]-1H-imidazol-4-yl}-propionsäure,
3-(6-Amino-pyridin-3-yl)-2-[1-(5-cyclopentyl-[1,3,4]thiadiazol-2-ylmethyl)-1 H-imidazol-4-yl]-propionsäure,
3-(6-Amino-pyridin-3-yl)-2-[1-(5-cyclobutyl-[1,3,4]thiadiazol-2-ylmethyl)-1H-imidazol-4-yl]-propionsäure,
3-(6-Amino-pyridin-3-yl)-2-[1-(5-cyclopropyl-isoxazol-3-ylmethyl)-1 H-imidazol-4-yl]-propionsäure,
3-(6-Amino-pyridin-3-yl)-2-[1-(5-cyclohexyl-isoxazol-3-ylmethyl)-1 H-imidazol-4-yl]-propionsäure,
3-(6-Amino-pyridin-3-yl)-2-[1-(5-cyclohexyl-[1,3,4]thiadiazol-2-ylmethyl)-1H-imidazol-4-yl]-propionsäure,
3-(6-Amino-pyridin-3-yl)-2-[1-(5-cyclobutyl-isoxazol-3-ylmethyl)-1 H-imidazol-4-yl]-propionsäure,
3-(6-Amino-pyridin-3-yl)-2-{1-[5-(4-fluor-phenyl)-isoxazol-3-ylmethyl]-1H-imidazol-4-yl}-propionsäure,
3-(6-Amino-pyridin-3-yl)-2-{1-[5-(4-benzyl-phenyl)-isoxazol-3-ylmethyl]-1H-imidazol-4-yl}-propionsäure,
3-(6-Amino-pyridin-3-yl)-2-{1-[5-(4-tert-butyl-phenyl)-isoxazol-3-ylmethyl]-1H-imidazol-4-yl}-propionsäure,
3-(6-Amino-pyridin-3-yl)-2-{1-[5-(4-tert-butyl-2,6-dimethyl-phenyl)-isoxazol-3-ylmethyl]-1H-imidazol-4-yl}-propionsäure,
3-(6-Amino-pyridin-3-yl)-2-{1-[5-(2-chlor-phenyl)-isoxazol-3-ylmethyl]-1H-imidazol-4-yl}-propionsäure,
3-(6-Amino-pyridin-3-yl)-2-{1-[5-(4-sec-butyl-phenyl)-isoxazol-3-ylmethyl]-1H-imidazol-4-yl}-propionsäure,
3-(6-Amino-pyridin-3-yl)-2-[1-(5-indan-5-yl-isoxazol-3-ylmethyl)-1 H-imidazol-4-yl]-propionsäure,
3-(6-Amino-pyridin-3-yl)-2-{1-[5-(4-cyclopentyl-phenyl)-isoxazol-3-ylmethyl]-1H-imidazol-4-yl}-propionsäure,
3-(6-Amino-pyridin-3-yl)-2-{1-[5-(4-isopropyl-phenyl)-isoxazol-3-ylmethyl]-1H-imidazol-4-yl}-propionsäure,
3-(6-Amino-pyridin-3-yl)-2-{1-[5-(4-butyl-phenyl)-isoxazol-3-ylmethyl]-1H-imidazol-4-yl}-propionsäure,
3-(6-Amino-pyridin-3-yl)-2-{1-[5-(4-cyclohexyl-phenyl)-isoxazol-3-ylmethyl]-1H-imidazol-4-yl}-propionsäure,
3-(6-Amino-pyridin-3-yl)-2-{1-[5-(5,6,7,8-tetrahydro-naphthalen-2-yl)-isoxazol-3-ylmethyl]-1H-imidazol-4-yl}-propionsäure,
3-(6-Amino-pyridin-3-yl)-2-{1-[5-(4-propyl-phenyl)-isoxazol-3-ylmethyl]-1H-imidazol-4-yl}-propionsäure,
3-(6-Amino-pyridin-3-yl)-2-{1-[5-(4-phenethyl-phenyl)-isoxazol-3-ylmethyl]-1H-imidazol-4-yl}-propionsäure,
3-(6-Amino-pyridin-3-yl)-2-{1-[5-(2,3-dihydro-benzo[1,4]dioxin-6-yl)-isoxazol-3-ylmethyl]-1H-imidazol-4-yl}-propionsäure,
(S)-3-(6-Amino-pyridin-3-yl)-2-{1-[5-(5-chlor-thiophen-2-yl)-isoxazol-3-ylmethyl]-1H-imidazol-4-yl}-propionsäureethylester,
(R)-3-(6-Amino-pyridin-3-yl)-2-{1-[5-(5-chlor-thiophen-2-yl)-isoxazol-3-ylmethyl]-1H-imidazol-4-yl}-propionsäureethylester,
(S)-3-(6-Amino-pyridin-3-yl)-2-{1-[5-(5-chlor-thiophen-2-yl)-isoxazol-3-ylmethyl]-1H-imidazol-4-yl}-propionsäure,
(R)-3-(6-Amino-pyridin-3-yl)-2-{1-[5-(5-chlor-thiophen-2-yl)-isoxazol-3-ylmethyl]-1H-imidazol-4-yl}-propionsäure,
3-(6-Amino-5-methyl-pyridin-3-yl)-2-[1-(5-cyclopropyl-[1,3,4]thiadiazol-2-ylmethyl)-1 H-imidazol-4-yl]-propionsäure,

3. Verfahren zur Herstellung der Verbindung der Formel I gemäß einem der Ansprüche 1 oder 2, **dadurch gekennzeichnet, dass** man
a) eine Verbindung der Formel III 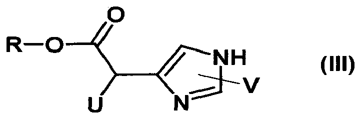 wobei R, U und V die in der Verbindung der Formel I gemäß Anspruch 1 genannten Bedeutungen haben,
mit einer Verbindung LG-W-X-Y, wobei W, X und Y die in der Verbindung der Formel I gemäß Anspruch 1 genannten Bedeutungen haben und LG eine Abgangsgruppe aus der Reihe -Cl, -Br, -I, -O-Tosyl oder -O-Mesyl darstellt, zu einer Verbindung der Formel I gemäß Anspruch I umsetzt, wobei R nicht Wasserstoffatom ist, oder
die Verbindung der Formel I gemäß Anspruch I, wobei R nicht Wasserstoffatom ist, durch Spaltung des Esters in eine Verbindung der Formel I umsetzt, wobei R Wasserstoffatom bedeutet, oder
b) eine nach dem Verfahren a) hergestellte Verbindung der Formel I, oder eine geeignete Vorstufe der Formel I, die aufgrund ihrer chemischen Struktur in enantiomeren Formen auftritt, durch Salzbildung mit enantiomerenreinen Säuren oder Basen, Chromatographie an chiralen Stationärphasen oder Derivatisierung mittels chiraler enantiomerenreiner Verbindungen wie Aminosäuren, Trennung der somit erhaltenen Diastereomeren, und Abspaltung der chiralen Hilfsgruppen in die reinen Enantiomeren auftrennt, oder
c) die nach den Verfahren a) oder b) hergestellte Verbindung der Formel I entweder in freier Form isoliert oder im Falle des Vorliegens von sauren oder basischen Gruppen in physiologisch verträgliche Salze umwandelt.

4. Arzneimittel, **gekennzeichnet durch** einen wirksamen Gehalt an mindestens einer Verbindung der Formel I gemäß einem der Ansprüche 1 oder 2 zusammen mit einem pharmazeutisch geeigneten und physiologisch verträglichen Trägerstoff, Zusatzstoff und/oder anderen Wirk- und Hilfsstoffen.

5. Verwendung der Verbindung der Formel I gemäß einem der Ansprüche 1 oder 2 zur Herstellung eines Arzneimittels zur Prophylaxe, Sekundärprevention und Therapie all solcher Erkrankungen, die die mit Thrombosen, Embolien, Hyperkoagulabilität oder fibrotischen Veränderungen einhergehen.

6. Verwendung gemäß Anspruch 5, **dadurch gekennzeichnet, dass** es sich um Myokardinfarkt, Angina pectoris und andere Formen des akuten Koronarsyndroms, den Schlaganfall, die peripher vaskulären Erkrankungen, die tiefe Venenthrombose, die Lungenembolie, embolische oder thrombotische Ereignisse bedingt durch kardiale Arrhythmien, kardiovaskuläre Ereignisse wie Restenose nach Revaskularisierung und Angioplastie und ähnlichen Eingriffen wie Stentimplantationen und Bypass-Operationen handelt, oder die Reduktion der Thrombosegefahr nach chirurgischen Eingriffen wie bei Knie- und Hüftgelenksoperationen, oder um die disseminierte intravaskuläre Koagulation, Sepsis und anderen intravaskulären Ereignissen, die mit einer Entzündung einhergehen, Atherosklerose, Diabetes und dem metabolischen Syndrom und deren Folgen, Tumorwachstum und Tumormetastasierung, entzündliche und degenerative Gelenkerkrankungen wie der rheumatoiden Arthritis und der Arthrose, Störungen des hämostatischen Systems wie Fibrinablagerungen, fibrotische Veränderungen der Lunge wie die chronische obstruktive Lungenerkrankung, das adult respiratory distress syndrom oder Fibrinablagerungen des Auges nach Augenoperationen oder Verhinderung und/oder Behandlung von Narbenbildung.

## Claims

1. A compound of the formula I 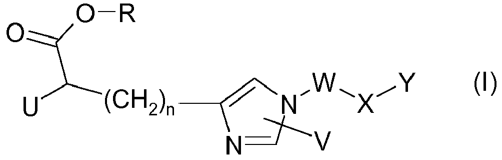 and/or any stereoisomeric form of the compound of the formula I and/or a mixture of these forms in any ratio and/or a physiologically acceptable salt of the compound of the formula I where U is -(C₁-C₄)-alkylene-Het-Z and where U and Z together are the radical 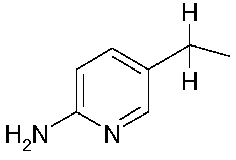 and the pyridyl moiety in the radical is unsubstituted or substituted by methyl or ethyl,
n is zero,
R is a hydrogen atom or -(C₁-C₄)-alkyl,
V is
1) a hydrogen atom,
2) -(C₁-C₃)-alkyl or
3) fluorine, chlorine or bromine,
W is -(C₁-C₃)-alkylene,
X is an aromatic five- to thirteen-membered heterocycle, where the heterocycle is selected from the group consisting of isoxazolyl, 1,2,3-oxadiazolyl, 1,2,4-oxadiazolyl, 1,2,5-oxadiazolyl, 1,3,4-oxadiazolyl, oxazolyl, 1,2,3-thiadiazolyl, 1,2,4-thiadiazolyl, 1,2,5-thiadiazolyl, 1,3,4-thiadiazolyl, thienopyridinyl or thienyl, and the heterocycle is unsubstituted or mono-, di- or trisubstituted independently by R1,
Y is
1) -(C₃-C₆)-cycloalkyl, where cycloalkyl is unsubstituted or mono-, di- or trisubstituted independently by R1,
2) -(C₆-C₁₄)-aryl, where aryl is selected from the group consisting of indanyl, naphthyl, phenyl or tetrahydronaphthalenyl, where aryl is unsubstituted or mono-, di- or trisubstituted independently by R1, or
3) a heterocycle, where the heterocycle is selected from the group consisting of 2,3-dihydrobenzo[1,4]dioxin, isoxazolyl, 1,2,3-oxadiazolyl, 1,2,4-oxadiazolyl, 1,2,5-oxadiazolyl, 1,3,4-oxadiazolyl, oxazolyl, 1,2,3-thiadiazolyl, 1,2,4-thiadiazolyl, 1,2,5-thiadiazolyl, 1,3,4-thiadiazolyl, thienopyridinyl or thienyl, and Het is unsubstituted or mono-, di- or trisubstituted independently by R1, and
R1 is fluorine, chlorine, bromine, -(C₁-C₄)-alkyl, -(C₀-C₄)-alkylene-phenyl, -O-CH₃, -O-(C₁-C₄)-alkylene-phenyl, where phenyl is unsubstituted or mono- or disubstituted by fluorine, chlorine, bromine or -O-(C₁-C₄)-alkyl, -(C₃-C₆)-cycloalkyl or - CF₃.

2. The compound of the formula I as claimed in claim 1, which is the compound
3-(6-aminopyridin-3-yl)-2-{1-[5-(5-chlorothiophen-2-yl)isoxazol-3-ylmethyl]-1H-imidazol-4-yl}propionic acid,
3-(6-aminopyridin-3-yl)-2-[1-(5-phenylisoxazol-3-ylmethyl)-1H-imidazol-4-yl]propionic acid,
3-(6-aminopyridin-3-yl)-2-{1-[5-(4-methoxyphenyl)isoxazol-3-ylmethyl]-1H-imidazol-4-yl}propionic acid,
3-(6-aminopyridin-3-yl)-2-{1-[5-(5-chlorothiophen-2-yl)-[1,3,4]thiadiazol-2-ylmethyl]-1H-imidazol-4-yl}propionic acid,
3-(6-aminopyridin-3-yl)-2-{1-[5-(4-chlorophenyl)-[1,3,4]thiadiazol-2-ylmethyl]-1H-imidazol-4-yl}propionic acid,
3-(6-aminopyridin-3-yl)-2-{1-[5-(4-trifluoromethylphenyl)-[1,2,4]oxadiazol-3-ylmethyl]-1H-imidazol-4-yl}propionic acid,
3-(6-aminopyridin-3-yl)-2-{1-[5-(3-trifluoromethylphenyl)-[1,2,4]oxadiazol-3-ylmethyl]-1H-imidazol-4-yl}propionic acid,
3-(6-aminopyridin-3-yl)-2-[1-(5-cyclopropyl-[1,3,4]thiadiazol-2-ylmethyl)-1H-imidazol-4-yl]propionic acid,
3-(6-aminopyridin-3-yl)-2-[1-(5-phenyl-[1,2,4]oxadiazol-3-ylmethyl)-1H-imidazol-4-yl]propionic acid,
3-(6-aminopyridin-3-yl)-2-{1-[3-(3,4-dimethoxyphenyl)-[1,2,4]oxadiazol-5-ylmethyl]-1H-imidazol-4-yl}propionic acid,
3-(6-aminopyridin-3-yl)-2-{1-[3-(4-methoxyphenyl)-[1,2,4]oxadiazol-5-ylmethyl]-1H-imidazol-4-yl}propionic acid,
3-(6-aminopyridin-3-yl)-2-{1-[3-(4'-isopropylphenyl)-[1,2,4]oxadiazol-5-ylmethyl]-1H-imidazol-4-yl}propionic acid,
3-(6-aminopyridin-3-yl)-2-{1-[3[(4'-tert-butylphenyl)-[1,2,4]oxadiazol-5-ylmethyl]-1H-imidazol-4-yl]propionic acid,
3-(6-aminopyridin-3-yl)-2-{1-[5-(3,4-dichlorophenyl)isoxazol-3-ylmethyl]-1H-imidazol-4-yl}propionic acid,
3-(6-aminopyridin-3-yl)-2-{1-[3-(4-methoxyphenyl)-[1,2,4]oxadiazol-5-ylmethyl]-1H-imidazol-4-yl}propionic acid,
3-(6-aminopyridin-3-yl)-2-[1-(5-phenyl-[1,3,4]thiadiazol-2-ylmethyl)-1H-imidazol-4-yl]propionic acid,
3-(6-aminopyridin-3-yl)-2-{1-[5-(4-methoxyphenyl)-[1,3,4]thiadiazol-2-ylmethyl]-1H-imidazol-4-yl}propionic acid,
3-(6-aminopyridin-3-yl)-2-{1-[5-(3,5-dimethylisoxazol-4-yl)-[1,2,4]oxadiazol-3-ylmethyl]-1H-imidazol-4-yl}propionic acid,
3-(6-aminopyridin-3-yl)-2-[1-(5-thiophen-2-ylisoxazol-3-ylmethyl)-1H-imidazol-4-yl]propionic acid,
3-(6-aminopyridin-3-yl)-2-(1-{3-[4-(4-chlorobenzyloxy)phenyl]-[1,2,4]oxadiazol-5-ylmethyl}-1H-imidazol-4-yl)propionic acid,
3-(6-aminopyridin-3-yl)-2-[1-(4-phenyl-5-trifluoromethylthiophen-2-ylmethyl)-1H-imidazol-4-yl]propionic acid,
3-(6-aminopyridin-3-yl)-2-{1-[5-(4-bromophenyl)isoxazol-3-ylmethyl]-1H-imidazol-4-yl}propionic acid,
3-(6-aminopyridin-3-yl)-2-[1-(5-p-tolylisoxazol-3-ylmethyl)-1H-imidazol-4-yl]propionic acid,
3-(6-aminopyridin-3-yl)-2-(1-{2-[5-(5-chlorothiophen-2-yl)isoxazol-3-yl]-ethyl}-1H-imidazol-4-yl)propionic acid,
3-(6-aminopyridin-3-yl)-2-{1-[5-(4-isobutylphenyl)isoxazol-3-ylmethyl]-1H-imidazol-4-yl}propionic acid,
3-(6-aminopyridin-3-yl)-2-[1-(5-cyclopentyl-[1,3,4]thiadiazol-2-ylmethyl)-1H-imidazol-4-yl]propionic acid,
3-(6-aminopyridin-3-yl)-2-[1-(5-cyclobutyl-[1,3,4]thiadiazol-2-ylmethyl)-1H-imidazol-4-yl]propionic acid,
3-(6-aminopyridin-3-yl)-2-[1-(5-cyclopropylisoxazol-3-ylmethyl)-1H-imidazol-4-yl]propionic acid,
3-(6-aminopyridin-3-yl)-2-[1-(5-cyclohexylisoxazol-3-ylmethyl)-1H-imidazol-4-yl]propionic acid,
3-(6-aminopyridin-3-yl)-2-[1-(5-cyclohexyl-[1,3,4]thiadiazol-2-ylmethyl)-1H-imidazol-4-yl]propionic acid,
3-(6-aminopyridin-3-yl)-2-[1-(5-cyclobutylisoxazol-3-ylmethyl)-1H-imidazol-4-yl]propionic acid,
3-(6-aminopyridin-3-yl)-2-{1-[5-(4-fluorophenyl)isoxazol-3-ylmethyl]-1H-imidazol-4-yl}propionic acid,
3-(6-aminopyridin-3-yl)-2-{1-[5-(4-benzylphenyl)isoxazol-3-ylmethyl]-1H-imidazol-4-yl}propionic acid,
3-(6-aminopyridin-3-yl)-2-{1-[5-(4-tert-butylphenyl)isoxazol-3-ylmethyl]-1H-imidazol-4-yl}propionic acid,
3-(6-aminopyridin-3-yl)-2-{1-[5-(4-tert-butyl-2,6-dimethylphenyl)isoxazol-3-ylmethyl]-1H-imidazol-4-yl}propionic acid,
3-(6-aminopyridin-3-yl)-2-{1-[5-(2-chlorophenyl)isoxazol-3-ylmethyl]-1H-imidazol-4-yl}propionic acid,
3-(6-aminopyridin-3-yl)-2-{1-[5-(4-sec-butylphenyl)isoxazol-3-ylmethyl]-1H-imidazol-4-yl}propionic acid,
3-(6-aminopyridin-3-yl)-2-[1-(5-indan-5-ylisoxazol-3-ylmethyl)-1H-imidazol-4-yl]propionic acid,
3-(6-aminopyridin-3-yl)-2-{1-[5-(4-cyclopentylphenyl)isoxazol-3-ylmethyl]-1H-imidazol-4-yl}propionic acid,
3-(6-aminopyridin-3-yl)-2-{1-[5-(4-isopropylphenyl)isoxazol-3-ylmethyl]-1H-imidazol-4-yl}propionic acid,
3-(6-aminopyridin-3-yl)-2-{1-[5-(4-butylphenyl)isoxazol-3-ylmethyl]-1H-imidazol-4-yl}propionic acid,
3-(6-aminopyridin-3-yl)-2-{1-[5-(4-cyclohexylphenyl)isoxazol-3-ylmethyl]-1H-imidazol-4-yl}propionic acid,
3-(6-aminopyridin-3-yl)-2-{1-[5-(5,6,7,8-tetrahydronaphthalen-2-yl)isoxazol-3-ylmethyl]-1H-imidazol-4-yl}propionic acid,
3-(6-aminopyridin-3-yl)-2-{1-[5-(4-propylphenyl)isoxazol-3-ylmethyl]-1H-imidazol-4-yl}propionic acid,
3-(6-aminopyridin-3-yl)-2-{1-[5-(4-phenethylphenyl)isoxazol-3-ylmethyl]-1H-imidazol-4-yl}propionic acid,
3-(6-aminopyridin-3-yl)-2-{1-[5-(2,3-dihydrobenzo[1,4]dioxin-6-yl)isoxazol-3-ylmethyl]-1H-imidazol-4-yl}propionic acid,
ethyl (S)-3-(6-aminopyridin-3-yl)-2-{1-[5-(5-chlorothiophen-2-yl)isoxazol-3-ylmethyl]-1H-imidazol-4-yl}propionate,
ethyl (R)-3-(6-aminopyridin-3-yl)-2-{1-[5-(5-chlorothiophen-2-yl)isoxazol-3-ylmethyl]-1H-imidazol-4-yl}propionate,
(S)-3-(6-aminopyridin-3-yl)-2-{1-[5-(5-chlorothiophen-2-yl)isoxazol-3-ylmethyl]-1H-imidazol-4-yl}propionic acid,
(R)-3-(6-aminopyridin-3-yl)-2-{1-[5-(5-chlorothiophen-2-yl)isoxazol-3-ylmethyl]-1H-imidazol-4-yl}propionic acid,
3-(6-amino-5-methylpyridin-3-yl)-2-[1-(5-cyclopropyl-[1,3,4]thiadiazol-2-ylmethyl)-1H-imidazol-4-yl]propionic acid.

3. A process for preparing the compound of the formula I as claimed in either of claims 1 and 2, which comprises
a) reacting a compound of the formula III 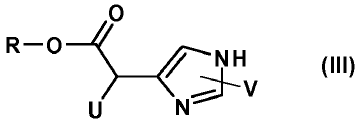 where R, U and V have the meanings given for the compound of the formula I as claimed in claim 1, with a compound LG-W-X-Y where W, X and Y have the meanings given for the compound of the formula I as claimed in claim 1 and LG is a leaving group from the group consisting of -Cl, -Br, -I, -0-tosyl or - O-mesyl to give a compound of the formula I as claimed in claim 1 where R is not a hydrogen atom,
or
converting the compound of the formula I according to claim 1 where R is not a hydrogen atom by cleavage of the ester into a compound of the formula I where R is a hydrogen atom, or
b) a compound of the formula I which has been prepared by process a) or a suitable precursor of the formula I which occurs owing to its chemical structure in enantiomeric forms being fractionated by salt formation with enantiopure acids or bases, chromatography on chiral stationary phases or derivatization using chiral enantiopure compounds such as amino acids, separation of the diastereomers obtained in this way, and elimination of the chiral auxiliary groups into the pure enantiomers, or
c) the compound of the formula I prepared by processes a) or b) being either isolated in free form or, in the case where acidic or basic groups are present, converted into physiologically tolerated salts.

4. A medicament having an effective content of at least one compound of the formula I as claimed in either of claims 1 and 2 together with a pharmaceutically suitable and physiologically tolerated carrier, additive and/or other active ingredients and excipients.

5. The use of the compound of the formula I as claimed in either of claims 1 and 2 for producing a medicament for the prophylaxis, secondary prevention and therapy of all disorders associated with thromboses, embolisms, hypercoagulability or fibrotic changes.

6. The use as claimed in claim 5, which is applied to myocardial infarction, angina pectoris and other types of acute coronary syndrome, stroke, peripheral vascular disorders, deep vein thrombosis, pulmonary embolism, embolic or thrombotic events caused by cardiac arrhythmias, cardiovascular events such as restenosis following revascularization and angioplasty and similar procedures such as stent implantations and bypass operations, or reduction of the risk of thrombosis following surgical procedures as in knee and hip joint operations, or disseminated intravascular coagulation, sepsis and other intravascular events which are associated with an inflammation, atherosclerosis, diabetes and the metabolic syndrome and its sequelae, tumor growth and tumor metastasis, inflammable and degenerative joint disorders such as rheumatoid arthritis and arthrosis, impairments of the hemostatic system such as fibrin deposits, fibrotic changes of the lung such as chronic obstructive lung disease, adult respiratory distress syndrome or fibrin deposits in the eye after eye operations or prevention and/or treatment of scar formation.

## Revendications

1. Composé de formule I 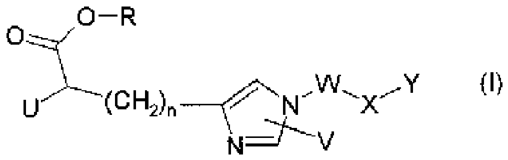 et/ou toutes formes stéréoisomères du composé de formule I et/ou tous mélanges de ces formes et tout rapport, et/ou sel physiologiquement acceptable du composé de formule I, dans laquelle
U représente un groupe -alkylène(C₁-C₄)-Het-Z et
U et Z représentant ensemble le radical 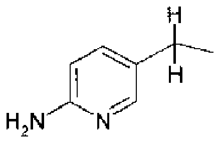 et le fragment pyridyle dans le radical étant non substitué ou substitué par méthyle ou éthyle,
n représente zéro,
R représente un atome d'hydrogène ou un groupe alkyle en C₁-C₄,
V représente
1) un atome d'hydrogène,
2) un groupe alkyle en C₁-C₃ ou
3) un atome de fluor, chlore ou brome,
W représente un groupe -alkylène(C₁-C₃),
X représente un hétérocycle aromatique à cinq à treize chaînons, l'hétérocycle étant choisi dans le groupe constitué par isoxazolyle, 1,2,3-oxadiazolyle, 1,2,4-oxadiazolyle, 1,2,5-oxadiazolyle, 1,3,4-oxadiazolyle, oxazolyle, 1,2,3-thiadiazolyle, 1,2,4-thiadiazolyle, 1,2,5-thiadiazolyle, 1,3,4-thiadiazolyle, thiénopyridine ou thiényle, et l'hétérocycle étant non substitué ou une, deux ou trois fois substitué, chaque fois indépendamment, par R1,
Y représente
1) un groupe -cycloalkyle(C₃-C₆), le groupe cycloalkyle étant non substitué ou une, deux ou trois fois substitué, chaque fois indépendamment, par R1,
2) un groupe -aryle(C₆-C₁₄), le groupe aryle étant choisi dans le groupe constitué par indanyle, naphtyle, phényle ou tétrahydronaphtalényle, le groupe aryle étant non substitué ou une, deux ou trois fois substitué, chaque fois indépendamment, par R1, ou
3) un hétérocycle, l'hétérocycle étant choisi dans le groupe constitué par 2,3-dihydrobenzo[1,4]dioxine, isoxazolyle, 1,2,3-oxadiazolyle, 1,2,4-oxadiazolyle, 1,2,5-oxadiazolyle, 1,3,4-oxadiazolyle, oxazolyle, 1,2,3-thiadiazolyle, 1,2,4-thiadiazolyle, 1,2,5-thiadiazolyle, 1,3,4-thiadiazolyle, thiénopyridine ou thiényle, et Het étant non substitué ou une, deux ou trois fois substitué, chaque fois indépendamment, par R1, et
R1 représente le fluor, le chlore, le brome, un groupe -alkyle(C₁-C₄), -alkylène(C₀-C₄)-phényle, -O-CH₃, -O-alkylène(C₁-C₄)-phényle, le groupe phényle étant non substitué ou une ou deux fois substitué par fluoro, chloro, bromo ou -O-alkyle(C₁-C₄), un groupe -cycloalkyle(C₃-C₆) ou -CF₃.

2. Composé de formule I selon la revendication 1, **caractérisé en ce qu'**il consiste en le composé acide 3-(6-amino-pyridin-3-yl)-2-{1-[5-(5-chlorothiophén-2-yl)-isoxazol-3-ylméthyl]-1H-imidazol-4-yl}propionique,
acide 3-(6-amino-pyridin-3-yl)-2-[1-(5-phényl-isoxazol-3-ylméthyl)-1H-imidazol-4-yl]-propionique,
acide 3-(6-amino-pyridin-3-yl)-2-{1-[5-(4-méthoxyphényl)-isoxazol-3-ylméthyl]-1H-imidazol-4-yl}propionique,
acide 3-(6-amino-pyridin-3-yl)-2-{1-[5-(5-chlorothiophén-2-yl)-[1,3,4]thiadiazol-2-ylméthyl]-1H-imidazol-4-yl}-propionique,
acide 3-(6-amino-pyridin-3-yl)-2-{1-[5-(4-chloro-phényl)-[1,3,4]thiadiazol-2-ylméthyl]-1H-imidazol-4-yl}-propionique,
acide 3-(6-amino-pyridin-3-yl)-2-{1-[5-(4-trifluorométhyl-phényl)-[1,2,4]oxadiazol-3-ylméthyl]-1H-imidazol-4-yl}-propionique,
acide 3-(6-amino-pyridin-3-yl)-2-{1-[5-(3-trifluorométhyl-phényl)-[1,2,4]oxadiazol-3-ylméthyl]-1H-imidazol-4-yl}-propionique,
acide 3-(6-amino-pyridin-3-yl)-2-[1-(5-cyclopropyl-[1,3,4]thiadiazol-2-ylméthyl)-1H-imidazol-4-yl]-propionique,
acide 3-(6-amino-pyridin-3-yl)-2-[1-(5-phényl-[1,2,4]oxadiazol-3-ylméthyl)-1H-imidazol-4-yl]-propionique,
acide 3-(6-amino-pyridin-3-yl)-2-{1-[3-(3,4-diméthoxy-phényl)-[1,2,4]oxadiazol-5-ylméthyl]-1H-imidazol-4-yl}-propionique,
acide 3-(6-amino-pyridin-3-yl)-2-{1-[3-(4-méthoxy-phényl)-[1,2,4]oxadiazol-5-ylméthyl]-1H-imidazol-4-yl}-propionique,
acide 3-(6-amino-pyridin-3-yl)-2-{1-[3-(4'-isopropyl-phényl)-[1,2,4]oxadiazol-5-ylméthyl]-1H-imidazol-4-yl}-propionique,
acide 3-(6-amino-pyridin-3-yl)-2-{1-[3-(4'-tert-butyl-phényl)-[1,2,4]oxadiazol-5-ylméthyl]-1H-imidazol-4-yl}-propionique,
acide 3-(6-amino-pyridin-3-yl)-2-{1-[5-(3,4-dichloro-phényl)-isoxazol-3-ylméthyl]-1H-imidazol-4-yl}-propionique,
acide 3-(6-amino-pyridin-3-yl)-2-{1-[3-(4-méthoxy-phényl)-[1,2,4]oxadiazol-5-ylméthyl]-1H-imidazol-4-yl}-propionique,
acide 3-(6-amino-pyridin-3-yl)-2-[1-(5-phényl-[1,3,4]thiadiazol-2-ylméthyl)-1H-imidazol-4-yl]-propionique,
acide 3-(6-amino-pyridin-3-yl)-2-{1-[5-(4-méthoxy-phényl)-[1,3,4]thiadiazol-2-ylméthyl]-1H-imidazol-4-yl}-propionique,
acide 3-(6-amino-pyridin-3-yl)-2-{1-[5-(3,5-diméthyl-isoxazol-4-yl)-[1,2,4]oxadiazol-3-ylméthyl]-1H-imidazol-4-yl}-propionique,
acide 3-(6-amino-pyridin-3-yl)-2-[1-(5-thiophén-2-yl-isoxazol-3-ylméthyl)-1H-imidazol-4-yl]-propionique, acide 3-(6-amino-pyridin-3-yl)-2-(1-{3-[4-(4-chloro-benzyloxy)-phényl]-[1,2,4]oxadiazol-5-ylméthyl}-1H-imidazol-4-yl)-propionique,
acide 3-(6-amino-pyridin-3-yl)-2-[1-(4-phényl-5-trifluorométhyl-thiophén-2-ylméthyl)-1H-imidazol-4-yl]-propionique,
acide 3-(6-amino-pyridin-3-yl)-2-{1-[5-(4-bromo-phényl)-isoxazol-3-ylméthyl]-1H-imidazol-4-yl}-propionique,
acide 3-(6-amino-pyridin-3-yl)-2-[1-(5-p-tolyl-isoxazol-3-ylméthyl)-1H-imidazol-4-yl]-propionique, acide 3-(6-amino-pyridin-3-yl)-2-(1-{2-[5-(5-chloro-thiophén-2-yl)-isoxazol-3-yl]-éthyl}-1H-imidazol-4-yl)-propionique,
acide 3-(6-amino-pyridin-3-yl)-2-{1-[5-4-isobutyl-phényl)-isoxazol-3-ylméthyl]-1H-imidazol-4-yl}-propionique,
acide 3-(6-amino-pyridin-3-yl)-2-[1-(5-cyclopentyl-[1,3,4]thiadiazol-2-ylméthyl)-1H-imidazol-4-yl]-propionique,
acide 3-(6-amino-pyridin-3-yl)-2-[1-(5-cyclobutyl-[1,3,4]thiadiazol-2-ylméthyl)-1H-imidazol-4-yl]-propionique,
acide 3-(6-amino-pyridin-3-yl)-2-[1-(5-cyclopropyl-isoxazol-3-ylméthyl)-1H-imidazol-4-yl]-propionique, acide 3-(6-amino-pyridin-3-yl)-2-[1-(5-cyclohexyl-isoxazol-3-ylméthyl)-1H-imidazol-4-yl]-propionique, acide 3-(6-amino-pyridin-3-yl)-2-[1-(5-cyclohexyl-[1,3,4]thiadiazol-2-ylméthyl)-1H-imidazol-4-yl]-propionique,
acide 3-(6-amino-pyridin-3-yl)-2-[1-(5-cyclobutyl-isoxazol-3-ylméthyl)-1H-imidazol-4-yl]-propionique, acide 3-(6-amino-pyridin-3-yl)-2-{1-[5-(4-fluoro-phényl)-isoxazol-3-ylméthyl]-1H-imidazol-4-yl}-propionique,
acide 3-(6-amino-pyridin-3-yl)-2-{1-[5-(4-benzyl-phényl)-isoxazol-3-ylméthyl]-1H-imidazol-4-yl}-propionique,
acide 3-(6-amino-pyridin-3-yl)-2-{1-[5-(4-tert-butyl-phényl)-isoxazol-3-ylméthyl]-1H-imidazol-4-yl}-propionique,
acide 3-(6-amino-pyridin-3-yl)-2-{1-[5-(4-tert-butyl-2,6-diméthyl-phényl)-isoxazol-3-ylméthyl]-1H-imidazol-4-yl}-propionique,
acide 3-(6-amino-pyridin-3-yl)-2-{1-[5-(2-chlorophényl)-isoxazol-3-ylméthyl]-1H-imidazol-4-yl}-propionique,
acide 3-(6-amino-pyridin-3-yl)-2-{1-[5-(4-sec-butyl-phényl)-isoxazol-3-ylméthyl]-1H-imidazol-4-yl}-propionique,
acide 3-(6-amino-pyridin-3-yl)-2-[1-(5-indan-5-yl-isoxazol-3-ylméthyl)-1H-imidazol-4-yl]-propionique, acide 3-(6-amino-pyridin-3-yl)-2-{1-[5-(4-cyclopentyl-phényl)-isoxazol-3-ylméthyl]-1H-imidazol-4-yl}-propionique,
acide 3-(6-amino-pyridin-3-yl)-2-{1-[5-(4-isopropyl-phényl)-isoxazol-3-ylméthyl]-1H-imidazol-4-yl}-propionique,
acide 3-(6-amino-pyridin-3-yl)-2-{1-[5-(4-butyl-phényl)-isoxazol-3-ylméthyl]-1H-imidazol-4-yl}-propionique,
acide 3-(6-amino-pyridin-3-yl)-2-{1-[5-(4-cyclohexyl-phényl)-isoxazol-3-ylméthyl]-1H-imidazol-4-yl}-propionique,
acide 3-(6-amino-pyridin-3-yl)-2-{1-[5-(5,6,7,8-tétrahydro-naphtalén-2-yl)-isoxazol-3-ylméthyl]-1H-imidazol-4-yl}-propionique,
acide 3-(6-amino-pyridin-3-yl)-2-{1-[5-(4-propyl-phényl)-isoxazol-3-ylméthyl]-1H-imidazol-4-yl}-propionique,
acide 3-(6-amino-pyridin-3-yl)-2-{1-[5-(4-phénéthyl-phényl)-isoxazol-3-ylméthyl]-1H-imidazol-4-yl}-propionique,
acide 3-(6-amino-pyridin-3-yl)-2-{1-[5-(2,3-dihydro-benzo[1,4]dioxin-6-yl)-isoxazol-3-ylméthyl]-1H-imidazol-4-yl}-propionique, (S)-3-(6-amino-pyridin-3-yl)-2-{1-[5-(5-chloro-thiophén-2-yl)-isoxazol-3-ylméthyl]-1H-imidazol-4-yl}-propionate d'éthyle, (R)-3-(6-amino-pyridin-3-yl)-2-{1-[5-(5-chloro-thiophén-2-yl)-isoxazol-3-ylméthyl]-1H-imidazol-4-yl}-propionate d'éthyle,
acide (S)-3-(6-amino-pyridin-3-yl)-2-{1-[5-(5-chloro-thiophén-2-yl)-isoxazol-3-ylméthyl]-1H-imidazol-4-yl}-propionique,
acide (R)-3-(6-amino-pyridin-3-yl)-2-{1-[5-(5-chloro-thiophén-2-yl)-isoxazol-3-ylméthyl]-1H-imidazol-4-yl}-propionique,
acide 3-(6-amino-5-méthyl-pyridin-3-yl)-2-[1-(5-cyclopropyl-[1,3,4]thiadiazol-2-ylméthyl)-1H-imidazol-4-yl]-propionique.

3. Procédé pour la préparation du composé de formule I selon l'une quelconque des revendications 1 et 2, **caractérisé en ce qu'**on fait réagir
a) un composé de formule III 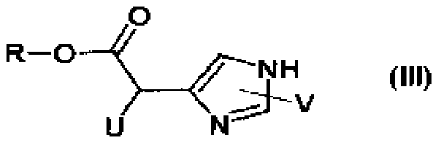 R, U et V ayant les significations indiquées dans le composé de formule I selon la revendication 1,
avec un composé LG-W-X-Y, W, X et Y ayant les significations indiquées dans le composé de formule I selon la revendication 1, et LG représentant un groupe partant choisi dans la série -Cl, -Br, -I, -O-tosyle ou -O-mésyle, pour aboutir à un composé de formule I selon la revendication 1, dans lequel R n'est pas un atome d'hydrogène, ou
on convertit le composé de formule I selon la revendication 1, dans lequel R n'est pas un atome d'hydrogène, par coupure de l'ester, en un composé de formule I dans lequel R représente un atome d'hydrogène, ou
b) on fractionne en les énantiomères purs un composé de formule I, préparé selon le procédé a), ou un précurseur approprié de la formule I, qui, en raison de sa structure chimique, apparaît sous des formes énantiomères, par salification avec des acides ou des bases sous forme d'énantiomères purs, chromatographie sur phases stationnaires chirales ou transformation en dérivés au moyen de composés chiraux sous forme d'énantiomères purs, tels que des acides aminés, séparation des diastéréoisomères ainsi obtenus, et élimination des groupes auxiliaires chiraux, ou
c) soit on isole sous forme libre le composé de formule I, préparé selon le procédé a) ou b), soit, dans le cas de la présence de groupes acides ou basiques, on le convertit en sels physiologiquement acceptables.

4. Médicament, **caractérisé par** une teneur efficace en au moins un composé de formule I selon l'une quelconque des revendications 1 et 2, conjointement avec un véhicule, additif et/ou d'autres principes actifs et adjuvants, pharmaceutiquement appropriés et physiologiquement acceptables.

5. Utilisation du composé de formule I selon l'une quelconque des revendications 1 et 2, pour la fabrication d'un médicament destiné à la prophylaxie, la prévention secondaire et la thérapie de toutes les maladies qui s'accompagnent de thromboses, d'embolies, d'hypercoagulabilité ou d'altérations fibreuses.

6. Utilisation selon la revendication 5, **caractérisée en ce qu'**il s'agit d'infarctus du myocarde, d'angine de poitrine et d'autres formes du syndrome coronarien aigu, de l'accident vasculaire cérébral, des maladies vasculaires périphériques, de la thrombose des veines profondes, de l'embolie pulmonaire, d'incidents emboliques ou thrombotiques dus à des arythmies cardiales, d'incidents cardiovasculaires tels que la resténose après revascularisation et angioplastie et interventions similaires telles qu'implantations de stents et opérations de pontage, ou pour la réduction du risque de thrombose après des interventions chirurgicales comme dans des opérations des articulations du genou et de la hanche, ou **en ce qu'**il s'agit de la coagulation intravasculaire disséminée, du sepsis, et d'autres incidents intravasculaires qui s'accompagnent d'une inflammation, de l'athérosclérose, du diabète et du syndrome métabolique et de leurs suites, de la croissance tumorale et de la dissémination de métastases tumorales, d'arthropathies inflammatoires et dégénératives telles que la polyarthrite rhumatoïde et l'arthrose, de troubles du système hémostatique tels que des dépôts de fibrine, d'altérations fibreuses du poumon telles que la pneumopathie obstructive chronique, le syndrome de détresse respiratoire de l'adulte ou de dépôts de fibrine dans l'oeil après des opérations des yeux ou pour éviter et/ou traiter la formation de cicatrices.
